# EUROPEAN PATENT APPLICATION

(11) **EP 3 875 470 A1**
(43) Date of publication of application: **08.09.2021**
(21) Application number: 19869281.6
(22) Date of filing: 04.10.2019
(51) Int. Cl.: C07K 14/005, A61K 39/29, A61K 39/385, A61P 35/00, A61P 31/12, A61K 39/00

(54) **CHIMERIC ANTIGEN WITH ENHANCED MULTI-IMMUNE FUNCTION THROUGH SPECIFIC BINDING TO TARGET CELL, AND USE THEREOF**

(30) Priority: 05.10.2018 KR 20180119294
(71) Applicant: RNAGENE Inc., Seoul 05855 (KR)
(72) Inventor: LEE, Woo Ghil, Seoul 05834 (KR)
(74) Representative: Rösler Rasch van der Heide & Partner
(86) International application number: PCT/KR2019/013065
(87) International publication number: WO 2020/071869

(57) **Abstract**

The present invention relates to a chimeric antigen, which binds specifically to target cells and enhances multiple immune functions, and the use thereof. Specifically, the present invention relates to: a chimeric antigen for inducing multiple immune functions wherein an immune response-inducing domain and a domain for inducing target cell-specific binding are fused to each other; a pharmaceutical composition for preventing or treating cancer, containing, as an active ingredient, the chimeric antigen for enhancing multiple immune functions; a pharmaceutical composition for preventing or treating infectious disease; a composition for enhancing immunity; and a vaccine composition.

<110>    Rnagene Inc.

<120>    Chimeric antigens that specifically bind to target cells to
         enhance multiple immune function and uses thereof

<130>    NPNC80346

<160>    28

<170>    KoPatentIn 3.0

<210>    1
<211>    429
<212>    PRT
<213>    Artificial Sequence

<220>
<223>    HBcAg polypeptide sequence


<400>    1
Met Glu Thr Thr Arg Pro Thr Arg Pro Ala Arg Gly Leu Glu Ala Thr
  1               5                  10                  15

Arg Pro Thr Arg Pro Leu Glu Ala Leu Glu Ala Leu Glu Ala Leu Glu
             20                  25                  30

Ala Leu Glu Ala Leu Glu Ala Leu Glu Ala Leu Glu Ala Thr Arg Pro
         35                  40                  45

Pro Arg Ala Met Glu Thr Val Ala Leu Thr His Arg Gly Leu Tyr Leu
     50                  55                  60

Glu Ala Met Glu Thr Ala Ser Asn Leu Glu Ala Ala Leu Ala Thr His
 65                  70                  75                  80

Arg Thr Arg Pro Val Ala Leu Gly Leu Tyr Ser Glu Arg Ala Ser Asn
                 85                  90                  95

Leu Glu Ala Gly Leu Ala Ala Ser Pro Pro Arg Ala Ala Leu Ala Ser
            100                 105                 110

Glu Arg Ala Arg Gly Gly Leu Ala Leu Glu Ala Val Ala Leu Val Ala
        115                 120                 125

Leu Gly Leu Tyr Thr Tyr Arg Val Ala Leu Ala Ser Asn Val Ala Leu
    130                 135                 140

Ala Ser Asn Met Glu Thr Gly Leu Tyr Leu Glu Ala Leu Tyr Ser Pro
145                 150                 155                 160

His Glu Ala Arg Gly Gly Leu Asn Leu Glu Ala Leu Glu Ala Thr Arg
                165                 170                 175

Pro Pro His Glu His Ile Ser Ile Leu Glu Ser Glu Arg Cys Tyr Ser
            180                 185                 190

Leu Glu Ala Thr His Arg Pro His Glu Gly Leu Tyr Ala Arg Gly Gly
        195                 200                 205

Leu Ala Thr His Arg Val Ala Leu Leu Glu Ala Gly Leu Ala Thr Tyr
    210                 215                 220

Arg Leu Glu Ala Val Ala Leu Ser Glu Arg Pro His Glu Gly Leu Tyr
225                 230                 235                 240

Val Ala Leu Thr Arg Pro Ile Leu Glu Ala Arg Gly Thr His Arg Pro
                245                 250                 255

Arg Ala Ala Leu Ala Pro Arg Ala Thr Tyr Arg Ala Arg Gly Pro Arg
            260                 265                 270

Ala Pro Arg Ala Ala Ser Asn Ala Leu Ala Pro Arg Ala Ile Leu Glu
        275                 280                 285

Leu Glu Ala Ser Glu Arg Thr His Arg Leu Glu Ala Pro Arg Ala Gly
    290                 295                 300

Leu Ala Thr His Arg Thr His Arg Val Ala Leu Val Ala Leu Ala Arg
305                 310                 315                 320

Gly Gly Leu Asn Ala Arg Gly Gly Leu Tyr Ala Arg Gly Ser Glu Arg
                325                 330                 335

Leu Glu Ala Ala Arg Gly Ala Arg Gly Ala Arg Gly Thr His Arg Pro
            340                 345                 350

Arg Ala Ser Glu Arg Pro Arg Ala Ala Arg Gly Ala Arg Gly Ala Arg
        355                 360                 365

Gly Ala Arg Gly Ser Glu Arg Gly Leu Asn Ser Glu Arg Pro Arg Ala
    370                 375                 380

Ala Arg Gly Ala Arg Gly Ala Arg Gly Ala Arg Gly Ser Glu Arg Leu
385                 390                 395                 400

Tyr Ser Ser Glu Arg Ala Arg Gly Ala Ser Pro Ser Glu Arg Gly Leu
                405                 410                 415

Asn Cys Tyr Ser Ser Glu Arg Thr His Arg Ser Glu Arg
            420                 425

<210>   2
<211>   429
<212>   DNA
<213>   Artificial Sequence

<220>
<223>   HBcAg polynucleotide sequence

<400>   2
atgtggtggc ggctctggtg gctgctcttg ctgctgttgc tgctgtggcc catggtcacc      60

ggcttgatga atcttgcgac ttgggtcggc tcaaaccttg aggacccggc gagtcgggaa     120

ctggtcgtgg gttatgtgaa tgtcaacatg ggcctcaaat tccggcagct cctctggttt     180

cacatttctt gccttacatt tggccgggaa acggttcttg aatacctggt atcattcggg     240

gtgtggattc ggacgcccgc tccatataga ccacctaacg cacccattct tagtacgttg     300

cctgagacga ctgtagttcg gcaacgagga cggtctctca gacgcagaac ccccagccca      360

agaagacgcc ggagccagtc tccccgccga aggcgatcaa agtctcgcga cagtcaatgc     420

tctactagt                                                          429

<210>  3
<211>  183
<212>  PRT
<213>  Artificial Sequence

<220>
<223>  HBeAg polypeptide sequence

<400>  3
Met Trp Trp Arg Leu Trp Trp Leu Leu Leu Leu Leu Leu Leu Leu Trp
1               5                   10                  15

Pro Met Val Thr Gly Lys Leu Cys Leu Gly Trp Leu Trp Gly Met Asp
            20                  25                  30

Ile Asp Pro Tyr Lys Glu Phe Gly Ala Ser Val Glu Leu Leu Ser Phe
        35                  40                  45

Leu Pro Ser Asp Phe Phe Pro Ser Ala Arg Asp Leu Leu Asp Thr Ala
    50                  55                  60

Ser Ala Leu Tyr Arg Glu Ala Leu Glu Ser Pro Glu His Cys Ser Pro
65                  70                  75                  80

His His Thr Ala Ile Arg Gln Ala Ile Leu Cys Trp Gly Glu Leu Met
                85                  90                  95

Asn Leu Ala Thr Trp Val Gly Ser Asn Leu Glu Asp Pro Ala Ser Arg
            100                 105                 110

Glu Leu Val Val Gly Tyr Val Asn Val Asn Met Gly Leu Lys Phe Arg
        115                 120                 125

Gln Leu Leu Trp Phe His Ile Ser Cys Leu Thr Phe Gly Arg Glu Thr
    130                 135                 140

Val Leu Glu Tyr Leu Val Ser Phe Gly Val Trp Ile Arg Thr Pro Ala
145                 150                 155                 160

Pro Tyr Arg Pro Pro Asn Ala Pro Ile Leu Ser Thr Leu Pro Glu Thr
                165                 170                 175

Thr Val Val Arg Arg Thr Ser
            180

<210>  4
<211>  549
<212>  DNA
<213>  Artificial Sequence

<220>
<223>  HBeAg polynucleotide sequence

<400>  4
atgtggtggc ggctctggtg gctgctcttg ctgctgttgc tgctgtggcc catggtcacc      60

ggcaagctgt gccttgggtg gctttggggc atggacattg acccgtataa agaatttgga 120

gcttctgtgg agttactctc ttttttgcct tctgacttct ttccttctgc tcgggatctc 180

ctcgacaccg cctctgctct gtatcgggag gccttagagt ctccagaaca ttgttcacct 240

caccatacag caatcaggca agctattctg tgttggggtg agttgatgaa tctggccacc 300

tgggtgggaa gtaatttgga agacccagca tccagggaat tagtagtcgg ctatgtcaat 360

gttaatatgg gcctaaaatt cagacaacta ttgtggtttc acatttcctg tcttactttt 420

ggaagagaaa ctgtgctgga gtatttggtg tcttttggag tgtggattcg cactcctgct 480

ccttacagac caccaaatgc ccctatctta tcaacacttc cggaaactac tgttgttaga 540

aggactagt 549

<210> 5
<211> 226
<212> PRT
<213> Artificial Sequence

<220>
<223> HBsAg polypeptide sequence

<400> 5
Met Glu Asn Thr Thr Ser Gly Phe Leu Gly Pro Leu Leu Val Leu Gln
1               5                   10                  15

Ala Gly Phe Phe Leu Leu Thr Arg Ile Leu Thr Ile Pro Gln Ser Leu
            20                  25                  30

Asp Ser Trp Trp Thr Ser Leu Asn Phe Leu Gly Gly Ala Pro Thr Cys
        35                  40                  45

Pro Gly Gln Asn Ser Gln Ser Pro Thr Ser Asn His Ser Pro Thr Ser
    50                  55                  60

Cys Pro Pro Ile Cys Pro Gly Tyr Arg Trp Met Cys Leu Arg Arg Phe
65                  70                  75                  80

Ile Ile Phe Leu Phe Ile Leu Leu Leu Cys Leu Ile Phe Leu Leu Val
                85                  90                  95

Leu Leu Asp Tyr Gln Gly Met Leu Pro Val Cys Pro Leu Leu Pro Gly
            100                 105                 110

Thr Ser Thr Thr Ser Thr Gly Pro Cys Lys Thr Cys Thr Ile Pro Ala
        115                 120                 125

Gln Gly Thr Ser Met Phe Pro Ser Cys Cys Cys Thr Lys Pro Ser Asp
    130                 135                 140

Gly Asn Cys Thr Cys Ile Pro Ile Pro Ser Ser Trp Ala Phe Ala Arg
145                 150                 155                 160

Phe Leu Trp Glu Trp Ala Ser Val Arg Phe Ser Trp Leu Ser Leu Leu
                165                 170                 175

Val Pro Phe Val Gln Trp Phe Val Gly Leu Ser Pro Thr Val Trp Leu

180                 185                 190

Ser Val Ile Trp Met Met Trp Tyr Trp Gly Pro Ser Leu Tyr Asn Ile
        195                 200                 205

Leu Asn Pro Phe Leu Pro Leu Leu Pro Ile Phe Phe Tyr Leu Trp Val
    210                 215                 220

Tyr Ile
225

<210>    6
<211>    678
<212>    DNA
<213>    Artificial Sequence

<220>
<223>    HBsAg polynucleotide sequence

<400>    6
atggagaaca caacatcagg attcctagga cccctgctcg tgttacaggc ggggtttttc      60

ttgttgacaa gaatcctcac aataccacag agtctagact cgtggtggac ttctctcaat     120

tttctagggg gagcacccac gtgtcctggc caaaattcgc agtccccaac ctccaatcac     180

tcaccaacct cttgtcctcc aatttgtcct ggctatcgct ggatgtgtct gcggcgtttt     240

atcatattcc tcttcatcct gctgctatgc ctcatcttct tgttggttct tctggactat     300

caaggtatgt tgcccgtttg tcctctactt ccaggaacat caactaccag cacgggacca     360

tgcaagacct gcacgattcc tgctcaagga acctctatgt ttccctcttg ttgctgtaca     420

aaaccttcgg acggaaactg cacttgtatt cccatcccat catcttgggc tttcgcaaga     480

ttcctatggg agtgggcctc agtccgtttc tcctggctca gtttactagt gccatttgtt     540

cagtggttcg tagggctttc ccccactgtt tggctttcag ttatatggat gatgtggtat     600

tgggggccaa gtctgtacaa catcttgaat ccctttttac ctctattacc aattttcttt     660

tatctttggg tatacatt                                                   678

<210>    7
<211>    231
<212>    PRT
<213>    Artificial Sequence

<220>
<223>    PD-1 polypeptide sequence

<400>    7
Met Trp Trp Arg Leu Trp Trp Leu Leu Leu Leu Leu Leu Leu Leu Trp
  1               5                  10                  15

Pro Met Val Thr Gly Leu Glu Val Pro Asn Gly Pro Trp Arg Ser Leu
             20                  25                  30

Thr Phe Tyr Pro Ala Trp Leu Thr Val Ser Glu Gly Ala Asn Ala Thr
        35                  40                  45

Phe Thr Cys Ser Leu Ser Asn Trp Ser Glu Asp Leu His Val Val Trp
    50                  55                  60

His Leu Glu Ser Pro Ser Gly Gln Thr Glu Thr Gln Ala Ala Phe Cys
65                  70                  75                  80

Asn Gly Leu Ser Gln Pro Val Gln Asp Ala Arg Phe Gln Ile Ile Gln
                85                  90                  95

Leu Pro Asn Arg His Asp Phe His Met Asn Ile Leu Asp Thr Arg Arg
            100                 105                 110

Asn Asp Ser Gly Ile Tyr Val Cys Gly Val Ile Ser Leu His Pro Lys
        115                 120                 125

Ile Lys Ile Glu Glu Ser Pro Gly Ala Glu Leu Val Val Thr Glu Arg
    130                 135                 140

Ile Gly Ala Gly Ser Lys Asp Asp Thr Leu Lys Glu Glu Pro Ser Ala
145                 150                 155                 160

Ala Pro Val Pro Ser Val Ala Tyr Glu Glu Leu Asp Phe Gln Gly Arg
                165                 170                 175

Glu Lys Thr Pro Glu Leu Pro Thr Ala Cys Val His Thr Glu Tyr Ala
            180                 185                 190

Thr Ile Val Phe Thr Glu Gly Leu Gly Ala Ser Ala Met Gly Arg Arg
        195                 200                 205

Gly Ser Ala Asp Gly Leu Gln Gly Pro Arg Pro Pro Arg His Glu Asp
    210                 215                 220

Gly His Cys Ser Trp Pro Leu
225                 230


<210>    8
<211>    693
<212>    DNA
<213>    Artificial Sequence

<220>
<223>    PD-1 polynucleotide sequence


<400>    8
atgtggtggc ggctctggtg gctgctcttg ctgctgttgc tgctgtggcc catggtcacc      60

ggcctagaag tcccaaatgg gccctggaga agcttgacat tttatcctgc atggcttaca     120

gtaagtgagg gtgcaaacgc cactttcacc tgctcattga gtaattggtc agaagacctc     180

catgtcgttt ggcacctgga gtccccctca ggccagactg aaacccaggc cgcattttgt     240

aatggtctca gtcaacctgt gcaagatgcc aggtttcaaa tcattcagct ccccaatagg     300

catgatttcc acatgaatat cttggataca cgacgaaacg acagtggcat ttacgtgtgt     360

ggcgtcattt ctttgcatcc caagatcaag atagaggagt cccctggtgc tgagctcgtc     420

gtgacagagc ggatcggagc tgggtctaag gatgacactt tgaaggaaga gccatccgca 480

gcccccgtgc cctctgtcgc atacgaggaa ttggacttcc agggtcgaga aaaaacaccc 540

gagcttccaa ccgcttgtgt tcacaccgag tatgcaacca ttgtgttcac cgaaggcttg 600

ggggcttctg caatggggcg aaggggttcc gccgacggcc ttcaaggccc aaggcctccc 660

cgacacgaag atggacattg cagttggccc ctt 693

<210> 9
<211> 397
<212> PRT
<213> Artificial Sequence

<220>
<223> PD-1/HBcAg chimeric antigen polypeptide sequence

<400> 9
Met Trp Trp Arg Leu Trp Trp Leu Leu Leu Leu Leu Leu Leu Leu Trp
1               5                   10                  15

Pro Met Val Thr Gly Leu Glu Val Pro Asn Gly Pro Trp Arg Ser Leu
            20                  25                  30

Thr Phe Tyr Pro Ala Trp Leu Thr Val Ser Glu Gly Ala Asn Ala Thr
        35                  40                  45

Phe Thr Cys Ser Leu Ser Asn Trp Ser Glu Asp Leu His Val Val Trp
    50                  55                  60

His Leu Glu Ser Pro Ser Gly Gln Thr Glu Thr Gln Ala Ala Phe Cys
65                  70                  75                  80

Asn Gly Leu Ser Gln Pro Val Gln Asp Ala Arg Phe Gln Ile Ile Gln
                85                  90                  95

Leu Pro Asn Arg His Asp Phe His Met Asn Ile Leu Asp Thr Arg Arg
            100                 105                 110

Asn Asp Ser Gly Ile Tyr Val Cys Gly Val Ile Ser Leu His Pro Lys
        115                 120                 125

Ile Lys Ile Glu Glu Ser Pro Gly Ala Glu Leu Val Val Thr Glu Arg
    130                 135                 140

Ile Gly Ala Gly Ser Lys Asp Asp Thr Leu Lys Glu Glu Pro Ser Ala
145                 150                 155                 160

Ala Pro Val Pro Ser Val Ala Tyr Glu Glu Leu Asp Phe Gln Gly Arg
                165                 170                 175

Glu Lys Thr Pro Glu Leu Pro Thr Ala Cys Val His Thr Glu Tyr Ala
            180                 185                 190

Thr Ile Val Phe Thr Glu Gly Leu Gly Ala Ser Ala Met Gly Arg Arg
        195                 200                 205

Gly Ser Ala Asp Gly Leu Gln Gly Pro Arg Pro Pro Arg His Glu Asp
    210                 215                 220

Gly His Cys Ser Trp Pro Leu Ala Glu Ala Ala Ala Lys Glu Ala Ala
225                 230                 235                 240

Ala Lys Glu Ala Ala Ala Lys Glu Ala Ala Ala Lys Ala Leu Glu Ala
                245                 250                 255

Glu Ala Ala Ala Lys Glu Ala Ala Ala Lys Glu Ala Ala Ala Lys Glu
            260                 265                 270

Ala Ala Ala Lys Ala Leu Met Asn Leu Ala Thr Trp Val Gly Ser Asn
        275                 280                 285

Leu Glu Asp Pro Ala Ser Arg Glu Leu Val Val Gly Tyr Val Asn Val
    290                 295                 300

Asn Met Gly Leu Lys Phe Arg Gln Leu Leu Trp Phe His Ile Ser Cys
305                 310                 315                 320

Leu Thr Phe Gly Arg Glu Thr Val Leu Glu Tyr Leu Val Ser Phe Gly
                325                 330                 335

Val Trp Ile Arg Thr Pro Ala Pro Tyr Arg Pro Pro Asn Ala Pro Ile
            340                 345                 350

Leu Ser Thr Leu Pro Glu Thr Thr Val Val Arg Gln Arg Gly Arg Ser
        355                 360                 365

Leu Arg Arg Arg Thr Pro Ser Pro Arg Arg Arg Arg Ser Gln Ser Pro
    370                 375                 380

Arg Arg Arg Arg Ser Lys Ser Arg Asp Ser Gln Cys Ser
385                 390                 395


<210>    10
<211>    1191
<212>    DNA
<213>    Artificial Sequence

<220>
<223>    PD-1/HBcAg chimeric antigen polynucleotide sequence


<400>    10
atgtggtggc ggctctggtg gctgctcttg ctgctgttgc tgctgtggcc catggtcacc      60

ggcctagaag tcccaaatgg gccctggaga agcttgacat tttatcctgc atggcttaca     120

gtaagtgagg gtgcaaacgc cactttcacc tgctcattga gtaattggtc agaagacctc     180

catgtcgttt ggcacctgga gtccccctca ggccagactg aaacccaggc cgcattttgt     240

aatggtctca gtcaacctgt gcaagatgcc aggtttcaaa tcattcagct ccccaatagg     300

catgatttcc acatgaatat cttggataca cgacgaaacg acagtggcat ttacgtgtgt     360

ggcgtcattt ctttgcatcc caagatcaag atagaggagt cccctggtgc tgagctcgtc     420

gtgacagagc ggatcggagc tgggtctaag gatgacactt tgaaggaaga gccatccgca     480

gcccccgtgc cctctgtcgc atacgaggaa ttggacttcc agggtcgaga aaaaacaccc     540

gagcttccaa ccgcttgtgt tcacaccgag tatgcaacca ttgtgttcac cgaaggcttg 600

ggggcttctg caatggggcg aaggggttcc gccgacggcc ttcaaggccc aaggcctccc 660

cgacacgaag atggacattg cagttggccc cttgcagagg cagccgctaa agaggctgca 720

gctaaagaag cggcagccaa ggaagcagct gcaaaggcgt tggaagctga agctgcagca 780

aaggaggcag ctgcgaagga ggctgctgcc aaagaagccg ctgctaaagc tttgatgaat 840

cttgcgactt gggtcggctc aaaccttgag gacccggcga gtcgggaact ggtcgtgggt 900

tatgtgaatg tcaacatggg cctcaaattc cggcagctcc tctggtttca catttcttgc 960

cttacatttg gccgggaaac ggttcttgaa tacctggtat cattcggggt gtggattcgg 1020

acgcccgctc catatagacc acctaacgca cccattctta gtacgttgcc tgagacgact 1080

gtagttcggc aacgaggacg gtctctcaga cgcagaaccc ccagcccaag aagacgccgg 1140

agccagtctc cccgccgaag gcgatcaaag tctcgcgaca gtcaatgctc t 1191

<210> 11
<211> 413
<212> PRT
<213> Artificial Sequence

<220>
<223> PD-1/HBeAg chimeric antigen polypeptide sequence

<400> 11
Met Gly Val Lys Val Leu Phe Ala Leu Ile Cys Ile Ala Val Ala Glu
1               5                   10                  15

Ala Leu Glu Val Pro Asn Gly Pro Trp Arg Ser Leu Thr Phe Tyr Pro
            20                  25                  30

Ala Trp Leu Thr Val Ser Glu Gly Ala Asn Ala Thr Phe Thr Cys Ser
        35                  40                  45

Leu Ser Asn Trp Ser Glu Asp Leu His Val Val Trp His Leu Glu Ser
    50                  55                  60

Pro Ser Gly Gln Thr Glu Thr Gln Ala Ala Phe Cys Asn Gly Leu Ser
65                  70                  75                  80

Gln Pro Val Gln Asp Ala Arg Phe Gln Ile Ile Gln Leu Pro Asn Arg
                85                  90                  95

His Asp Phe His Met Asn Ile Leu Asp Thr Arg Arg Asn Asp Ser Gly
            100                 105                 110

Ile Tyr Val Cys Gly Val Ile Ser Leu His Pro Lys Ile Lys Ile Glu
        115                 120                 125

Glu Ser Pro Gly Ala Glu Leu Val Val Thr Glu Arg Ile Gly Ala Gly
    130                 135                 140

Ser Lys Asp Asp Thr Leu Lys Glu Glu Pro Ser Ala Ala Pro Val Pro
145                 150                 155                 160

Ser Val Ala Tyr Glu Glu Leu Asp Phe Gln Gly Arg Glu Lys Thr Pro
                165                 170                 175

Glu Leu Pro Thr Ala Cys Val His Thr Glu Tyr Ala Thr Ile Val Phe
            180                 185                 190

Thr Glu Gly Leu Gly Ala Ser Ala Met Gly Arg Arg Gly Ser Ala Asp
        195                 200                 205

Gly Leu Gln Gly Pro Arg Pro Pro Arg His Glu Asp Gly His Cys Ser
    210                 215                 220

Trp Pro Leu Gly Ser Gly Gly Gly Gly Ser Gly Gly Gly Gly Ser Gly
225                 230                 235                 240

Gly Gly Gly Ser Gly Gly Gly Gly Ser Gly Ser Lys Leu Cys Leu Gly
                245                 250                 255

Trp Leu Trp Gly Met Asp Ile Asp Pro Tyr Lys Glu Phe Gly Ala Ser
            260                 265                 270

Val Glu Leu Leu Ser Phe Leu Pro Ser Asp Phe Phe Pro Ser Ala Arg
        275                 280                 285

Asp Leu Leu Asp Thr Ala Ser Ala Leu Tyr Arg Glu Ala Leu Glu Ser
    290                 295                 300

Pro Glu His Cys Ser Pro His His Thr Ala Ile Arg Gln Ala Ile Leu
305                 310                 315                 320

Cys Trp Gly Glu Leu Met Asn Leu Ala Thr Trp Val Gly Ser Asn Leu
                325                 330                 335

Glu Asp Pro Ala Ser Arg Glu Leu Val Val Gly Tyr Val Asn Val Asn
            340                 345                 350

Met Gly Leu Lys Phe Arg Gln Leu Leu Trp Phe His Ile Ser Cys Leu
        355                 360                 365

Thr Phe Gly Arg Glu Thr Val Leu Glu Tyr Leu Val Ser Phe Gly Val
    370                 375                 380

Trp Ile Arg Thr Pro Ala Pro Tyr Arg Pro Pro Asn Ala Pro Ile Leu
385                 390                 395                 400

Ser Thr Leu Pro Glu Thr Thr Val Val Arg Arg Thr Ser
                405                 410


<210>    12
<211>    1239
<212>    DNA
<213>    Artificial Sequence

<220>
<223>    PD-1/HBeAg  chimeric antigen polynucleotide sequence


<400>    12
atgggagtga aagttctttt tgcccttatt tgtattgctg tggccgaggc cctagaagtc        60

ccaaatgggc cctggagaag cttgacattt tatcctgcat ggcttacagt aagtgagggt       120

gcaaacgcca ctttcacctg ctcattgagt aattggtcag aagacctcca tgtcgtttgg 180

cacctggagt ccccctcagg ccagactgaa acccaggccg cattttgtaa tggtctcagt 240

caacctgtgc aagatgccag gtttcaaatc attcagctcc ccaataggca tgatttccac 300

atgaatatct tggatacacg acgaaacgac agtggcattt acgtgtgtgg cgtcatttct 360

ttgcatccca agatcaagat agaggagtcc cctggtgctg agctcgtcgt gacagagcgg 420

atcggagctg ggtctaagga tgacactttg aaggaagagc catccgcagc ccccgtgccc 480

tctgtcgcat acgaggaatt ggacttccag ggtcgagaaa aaacacccga gcttccaacc 540

gcttgtgttc acaccgagta tgcaaccatt gtgttcaccg aaggcttggg ggcttctgca 600

atggggcgaa ggggttccgc cgacggcctt caaggcccaa ggcctccccg acacgaagat 660

ggacattgca gttggcccct tggatcagga ggtggtggtt cagggggagg agggagcggc 720

ggaggaggtt caggtggagg tggatctggt tcaaagctgt gccttgggtg gctttggggc 780

atggacattg acccgtataa agaatttgga gcttctgtgg agttactctc ttttttgcct 840

tctgacttct ttccttctgc tcgggatctc ctcgacaccg cctctgctct gtatcgggag 900

gccttagagt ctccagaaca ttgttcacct caccatacag caatcaggca agctattctg 960

tgttggggtg agttgatgaa tctggccacc tgggtgggaa gtaatttgga agacccagca 1020

tccagggaat tagtagtcgg ctatgtcaat gttaatatgg gcctaaaatt cagacaacta 1080

ttgtggtttc acatttcctg tcttactttt ggaagagaaa ctgtgctgga gtatttggtg 1140

tcttttggag tgtggattcg cactcctgct ccttacagac caccaaatgc ccctatctta 1200

tcaacacttc cggaaactac tgttgttaga aggactagt 1239

<210> 13
<211> 691
<212> DNA
<213> Artificial Sequence

<220>
<223> HBcAg IVT mRNA coding DNA template sequence

<400> 13
agatcttaat acgactcact atagggaaat aagagagaaa agaagagtaa gaagaaatat 60

aagagaattc gccaccatgt ggtggcggct ctggtggctg ctcttgctgc tgttgctgct 120

gtggcccatg gtcaccggct tgatgaatct tgcgacttgg gtcggctcaa accttgagga 180

cccggcgagt cgggaactgg tcgtgggtta tgtgaatgtc aacatgggcc tcaaattccg 240

gcagctcctc tggtttcaca tttcttgcct tacatttggc cgggaaacgg ttcttgaata 300

cctggtatca ttcggggtgt ggattcggac gcccgctcca tatagaccac ctaacgcacc 360

cattcttagt acgttgcctg agacgactgt agttcggcaa cgaggacggt ctctcagacg 420

cagaaccccc agcccaagaa gacgccggag ccagtctccc cgccgaaggc gatcaaagtc 480

tcgcgacagt caatgctcta ctagttagtc tagataacta gggatccgct gccttctgcg 540

gggcttgcct tctggccatg cccttcttct ctcccttgca cctgtacctc ttggtctttg 600

aataaagcct gagtaggaag aatattaaaa aaaaaaaaaa aaaaaaaaaa aaaaaaaaga 660

aaaaaaaaaa aaaaaaaaaa aaaaaaaaaa a 691

<210> 14
<211> 811
<212> DNA
<213> Artificial Sequence

<220>
<223> HBeAg IVT mRNA coding DNA template sequence

<400> 14
agatcttaat acgactcact atagggaaat aagagagaaa agaagagtaa gaagaaatat 60

aagagaattc gccaccatgt ggtggcggct ctggtggctg ctcttgctgc tgttgctgct 120

gtggcccatg gtcaccggca agctgtgcct tgggtggctt tggggcatgg acattgaccc 180

gtataaagaa tttggagctt ctgtggagtt actctctttt ttgccttctg acttctttcc 240

ttctgctcgg gatctcctcg acaccgcctc tgctctgtat cgggaggcct tagagtctcc 300

agaacattgt tcacctcacc atacagcaat caggcaagct attctgtgtt ggggtgagtt 360

gatgaatctg gccacctggg tgggaagtaa tttggaagac ccagcatcca gggaattagt 420

agtcggctat gtcaatgtta atatgggcct aaaattcaga caactattgt ggtttcacat 480

ttcctgtctt acttttggaa gagaaactgt gctggagtat ttggtgtctt ttggagtgtg 540

gattcgcact cctgctcctt acagaccacc aaatgcccct atcttatcaa cacttccgga 600

aactactgtt gttagaagga ctagttagtc tagataacta gggatccgct gccttctgcg 660

gggcttgcct tctggccatg cccttcttct ctcccttgca cctgtacctc ttggtctttg 720

aataaagcct gagtaggaag aatattaaaa aaaaaaaaaa aaaaaaaaaa aaaaaaaaga 780

aaaaaaaaaa aaaaaaaaaa aaaaaaaaaa a 811

<210> 15
<211> 927
<212> DNA
<213> Artificial Sequence

<220>
<223> HBsAg IVT mRNA coding DNA template sequence

<400> 15
agatcttaat acgactcact atagggaaat aagagagaaa agaagagtaa gaagaaatat 60

aagagaattc gccaccatgg agaacacaac atcaggattc ctaggacccc tgctcgtgtt 120

acaggcgggg tttttcttgt tgacaagaat cctcacaata ccacagagtc tagactcgtg 180

gtggacttct ctcaattttc tagggggagc acccacgtgt cctggccaaa attcgcagtc 240

cccaacctcc aatcactcac caacctcttg tcctccaatt tgtcctggct atcgctggat 300

gtgtctgcgg cgttttatca tattcctctt catcctgctg ctatgcctca tcttcttgtt 360

ggttcttctg gactatcaag gtatgttgcc cgtttgtcct ctacttccag gaacatcaac 420

taccagcacg ggaccatgca agacctgcac gattcctgct caaggaacct ctatgtttcc 480

ctcttgttgc tgtacaaaac cttcggacgg aaactgcact tgtattccca tcccatcatc 540

ttgggctttc gcaagattcc tatgggagtg ggcctcagtc cgtttctcct ggctcagttt 600

actagtgcca tttgttcagt ggttcgtagg gctttccccc actgtttggc tttcagttat 660

atggatgatg tggtattggg ggccaagtct gtacaacatc ttgaatccct ttttacctct 720

attaccaatt ttcttttatc tttgggtata catttaagga tccgctgcct tctgcggggc 780

ttgccttctg gccatgccct tcttctctcc cttgcacctg tacctcttgg tctttgaata 840

aagcctgagt aggaagaata ttaaaaaaaa aaaaaaaaaa aaaaaaaaaa aaaagaaaaa 900

aaaaaaaaaa aaaaaaaaaa aaaaaaa 927

<210> 16
<211> 955
<212> DNA
<213> Artificial Sequence

<220>
<223> PD-1 IVT mRNA coding DNA template sequence

<400> 16
agatcttaat acgactcact atagggaaat aagagagaaa agaagagtaa gaagaaatat 60

aagagaattc gccaccatgt ggtggcggct ctggtggctg ctcttgctgc tgttgctgct 120

gtggcccatg gtcaccggcc tagaagtccc aaatgggccc tggagaagct tgacatttta 180

tcctgcatgg cttacagtaa gtgagggtgc aaacgccact ttcacctgct cattgagtaa 240

ttggtcagaa gacctccatg tcgtttggca cctggagtcc ccctcaggcc agactgaaac 300

ccaggccgca ttttgtaatg gtctcagtca acctgtgcaa gatgccaggt ttcaaatcat 360

tcagctcccc aataggcatg atttccacat gaatatcttg gatacacgac gaaacgacag 420

tggcatttac gtgtgtggcg tcatttcttt gcatcccaag atcaagatag aggagtcccc 480

tggtgctgag ctcgtcgtga cagagcggat cggagctggg tctaaggatg acactttgaa 540

ggaagagcca tccgcagccc ccgtgccctc tgtcgcatac gaggaattgg acttccaggg 600

tcgagaaaaa acacccgagc ttccaaccgc ttgtgttcac accgagtatg caaccattgt 660

gttcaccgaa ggcttggggg cttctgcaat ggggcgaagg ggttccgccg acggccttca 720

aggcccaagg cctccccgac acgaagatgg acattgcagt tggccccttt agtctagata 780

actagggatc cgctgccttc tgcggggctt gccttctggc catgcccttc ttctctccct 840

tgcacctgta cctcttggtc tttgaataaa gcctgagtag gaagaatatt aaaaaaaaaa 900

aaaaaaaaaa aaaaaaaaaa aagaaaaaaa aaaaaaaaaa aaaaaaaaaa aaaaa 955

<210> 17
<211> 1620
<212> DNA
<213> Artificial Sequence

<220>
<223> PD-1/HBcAg IVT mRNA coding DNA template sequence

<400> 17
agatcttaat acgactcact atagggaaat aagagagaaa agaagagtaa gaagaaatat 60

aagagaattc gccaccatgt ggtggcggct ctggtggctg ctcttgctgc tgttgctgct 120

gtggcccatg gtcaccggcc tagaagtccc aaatgggccc tggagaagct tgacatttta 180

tcctgcatgg cttacagtaa gtgagggtgc aaacgccact ttcacctgct cattgagtaa 240

ttggtcagaa gacctccatg tcgtttggca cctggagtcc ccctcaggcc agactgaaac 300

ccaggccgca ttttgtaatg gtctcagtca acctgtgcaa gatgccaggt ttcaaatcat 360

tcagctcccc aataggcatg atttccacat gaatatcttg gatacacgac gaaacgacag 420

tggcatttac gtgtgtggcg tcatttcttt gcatcccaag atcaagatag aggagtcccc 480

tggtgctgag ctcgtcgtga cagagcggat cggagctggg tctaaggatg acactttgaa 540

ggaagagcca tccgcagccc ccgtgccctc tgtcgcatac gaggaattgg acttccaggg 600

tcgagaaaaa acacccgagc ttccaaccgc ttgtgttcac accgagtatg caaccattgt 660

gttcaccgaa ggcttggggg cttctgcaat ggggcgaagg ggttccgccg acggccttca 720

aggcccaagg cctccccgac acgaagatgg acattgcagt tggcccattg gctcaggcgg 780

tggaggttca gggggcgggg gctccggtgg tggcggttct ggtggaggag gctctggtgg 840

aggaggctcc ggcggaggtg gttctggcgg tgggggtagc gggggagggg ggtccgggtc 900

tgacattgac ccgtataaag aatttggagc ttctgtggag ttactctctt ttttgccttc 960

tgacttcttt ccttctgctc gagatctcct cgacaccgcc tctgctctgt atcgggaggc 1020

cttagagtct ccagaacatt gttcacctca ccatacagca atcaggcaag ctattctgtg 1080

ttggggtgag ttgatgaatc tggccacctg ggtgggaagt aatttggagg acccagcatc 1140

cagggaatta gtagtcggct atgtcaatgt taatatgggc ctaaaattca gacaactatt 1200

gtggtttcac atttcctgtc ttacttttgg aagagaaact gtgctggagt atttggtgtc 1260

ttttggagtg tggattcgca ctcctgctcc ttacagacca ccaaatgccc ctatcttatc 1320

aacacttccg gaaactactg ttgttagaca acgaggcagg tcccttagaa gaagaactcc 1380

ctcgcctcgc agacgaaggt ctcaatcgcc gcgtcgcaga agatctaaat ctcgggactc 1440

tcaatgttaa ggatccgctg ccttctgcgg ggcttgcctt ctggccatgc ccttcttctc 1500

tcccttgcac ctgtacctct tggtctttga ataaagcctg agtaggaaga atattaaaaa 1560

aaaaaaaaaa aaaaaaaaaa aaaaaaagaa aaaaaaaaaa aaaaaaaaaa aaaaaaaaaa 1620

1620

<210> 18
<211> 1501
<212> DNA
<213> Artificial Sequence

<220>
<223> PD-1/HBeAg IVT mRNA coding DNA template sequence

<400> 18
agatcttaat acgactcact atagggaaat aagagagaaa agaagagtaa gaagaaatat 60

aagagaattc gccaccatgg gagtgaaagt tctttttgcc cttatttgta ttgctgtggc 120

cgaggcccta gaagtcccaa atgggccctg gagaagcttg acattttatc ctgcatggct 180

tacagtaagt gagggtgcaa acgccacttt cacctgctca ttgagtaatt ggtcagaaga 240

cctccatgtc gtttggcacc tggagtcccc ctcaggccag actgaaaccc aggccgcatt 300

ttgtaatggt ctcagtcaac ctgtgcaaga tgccaggttt caaatcattc agctccccaa 360

taggcatgat ttccacatga atatcttgga tacacgacga aacgacagtg gcatttacgt 420

gtgtggcgtc atttctttgc atcccaagat caagatagag gagtcccctg gtgctgagct 480

cgtcgtgaca gagcggatcg gagctgggtc taaggatgac actttgaagg aagagccatc 540

cgcagccccc gtgccctctg tcgcatacga ggaattggac ttccagggtc gagaaaaaac 600

acccgagctt ccaaccgctt gtgttcacac cgagtatgca accattgtgt tcaccgaagg 660

cttgggggct tctgcaatgg ggcgaagggg ttccgccgac ggccttcaag gcccaaggcc 720

tccccgacac gaagatggac attgcagttg gccccttgga tcaggaggtg gtggttcagg 780

gggaggaggg agcggcggag gaggttcagg tggaggtgga tctggttcaa agctgtgcct 840

tgggtggctt tggggcatgg acattgaccc gtataaagaa tttggagctt ctgtggagtt 900

actctctttt ttgccttctg acttctttcc ttctgctcgg gatctcctcg acaccgcctc 960

tgctctgtat cgggaggcct tagagtctcc agaacattgt tcacctcacc atacagcaat 1020

caggcaagct attctgtgtt ggggtgagtt gatgaatctg gccacctggg tgggaagtaa 1080

tttggaagac ccagcatcca gggaattagt agtcggctat gtcaatgtta atatgggcct 1140

aaaattcaga caactattgt ggtttcacat ttcctgtctt acttttggaa gagaaactgt 1200

gctggagtat ttggtgtctt ttggagtgtg gattcgcact cctgctcctt acagaccacc      1260

aaatgcccct atcttatcaa cacttccgga aactactgtt gttagaagga ctagttagtc      1320

tagataacta gggatccgct gccttctgcg gggcttgcct tctggccatg cccttcttct      1380

ctcccttgca cctgtacctc ttggtctttg aataaagcct gagtaggaag aatattaaaa      1440

aaaaaaaaaa aaaaaaaaaa aaaaaaaaga aaaaaaaaaa aaaaaaaaaa aaaaaaaaaa      1500

a                                                                      1501


<210>    19
<211>    46
<212>    PRT
<213>    Artificial Sequence

<220>
<223>    Linker-1 peptide sequence


<400>    19
Ala Glu Ala Ala Ala Lys Glu Ala Ala Ala Lys Glu Ala Ala Ala Lys
1               5                   10                  15

Glu Ala Ala Ala Lys Ala Leu Glu Ala Glu Ala Ala Ala Lys Glu Ala
            20                  25                  30

Ala Ala Lys Glu Ala Ala Ala Lys Glu Ala Ala Ala Lys Ala
        35                  40                  45



<210>    20
<211>    138
<212>    DNA
<213>    Artificial Sequence

<220>
<223>    Linker-1 polynucleotide sequence


<400>    20
gcagaggcag ccgctaaaga ggctgcagct aaagaagcgg cagccaagga agcagctgca      60

aaggcgttgg aagctgaagc tgcagcaaag gaggcagctg cgaaggaggc tgctgccaaa      120

gaagccgctg ctaaagct                                                    138


<210>    21
<211>    24
<212>    PRT
<213>    Artificial Sequence

<220>
<223>    Linker-2 peptide sequence


<400>    21
Gly Ser Gly Gly Gly Gly Ser Gly Gly Gly Gly Ser Gly Gly Gly Gly

1               5                   10                  15

Ser Gly Gly Gly Gly Ser Gly Ser
            20


<210>   22
<211>   72
<212>   DNA
<213>   Artificial Sequence

<220>
<223>   Linker-2 polynucleotide sequence


<400>   22
ggatcaggag gtggtggttc agggggagga gggagcggcg gaggaggttc aggtggaggt      60

ggatctggtt ca                                                          72


<210>   23
<211>   21
<212>   PRT
<213>   Artificial Sequence

<220>
<223>   Signal peptide-1 polypeptide sequence


<400>   23
Met Trp Trp Arg Leu Trp Trp Leu Leu Leu Leu Leu Leu Leu Leu Trp
1               5                   10                  15

Pro Met Val Thr Gly
            20


<210>   24
<211>   63
<212>   DNA
<213>   Artificial Sequence

<220>
<223>   Signal peptide-1 polynucleotide sequence


<400>   24
atgtggtggc ggctctggtg gctgctcttg ctgctgttgc tgctgtggcc catggtcacc      60

ggc                                                                    63


<210>   25
<211>   17
<212>   PRT
<213>   Artificial Sequence

<220>
<223>   Signal peptide-2 polypeptide sequence


<400>   25

Met Gly Val Lys Val Leu Phe Ala Leu Ile Cys Ile Ala Val Ala Glu
1               5                   10                  15

Ala

<210>    26
<211>    51
<212>    DNA
<213>    Artificial Sequence

<220>
<223>    Signal peptide-2 polynucleotide sequence

<400>    26
atgggagtga aagttctttt tgcccttatt tgtattgctg tggccgaggc c                51

<210>    27
<211>    34
<212>    DNA
<213>    Artificial Sequence

<220>
<223>    primer1 sequence

<400>    27
agatcttaat acgactcact atagggaaat aaga                                   34

<210>    28
<211>    99
<212>    DNA
<213>    Artificial Sequence

<220>
<223>    primer2 sequence

<400>    28
tttttttttt tttttttttt tttttttttt ttcttttttt tttttttttt tttttttttt       60

tttttaatat tcttcctact caggctttat tcaaagacc                              99

## Description

### [Technical Field]

The present invention relates to a chimeric antigen, which binds specifically to target cells and enhances multiple immune functions, and the use thereof.

### [Background Art]

Immunity refers to the body's ability to prevent the invasion of external pathogens such as bacteria or viruses or to recognize and resist invading pathogens. Immunity includes innate immunity, which is the natural ability of every person, and acquired immunity which is acquired after birth. In a general sense, immunity refers to acquired immunity. Unlike innate immunity, also known as non-specific immunity, acquired immunity, also known as specific immunity, is a defense system that recognizes the type of invading pathogen and responds thereto, and lymphocytes and antibodies play an important role therein. In other words, B lymphocytes and T lymphocytes circulating in the body are responsible for acquired immunity. They have on their surface receptors that recognize specific antigens, and thus when they recognize the antigen, they activate humoral immunity and cellular immunity, respectively.

Antigens and antibodies are involved in acquired immunity, in which the antigens are substances that are recognized as non-self, such as pathogens, foreign substances, and organ tissues of other species, and cause an acquired immune response, and the antibodies are substances produced by B lymphocytes to fight against specific invading antigens, and eliminate pathogenicity by binding to specific antigens. In addition, the antibodies have the function of remembering the type of pathogen that invaded the body, and thus when the same pathogen invades a second time, the antibodies can effectively fight against the pathogen. Acquired immunity occurs after a pathogen completely invades the body, and acquired immunity is also called "secondary defense" because it occurs after innate immunity. Acquired immunity includes cellular immunity and humoral immunity. T lymphocytes and B lymphocytes have receptors on their surface that recognize specific antigens, and when they recognize antigens, they activate cellular immunity and humoral immunity, respectively. In the former (cellular immunity), cytotoxic T lymphocytes that recognize antigens directly attack and remove cells infected with pathogens or cancer cells, whereas, in the latter (humoral immunity), helper T lymphocytes that recognize antigens stimulate B lymphocytes to differentiate and proliferate into plasma cells that secrete antibodies and memory cells that remember antigens. The resulting generation of memory cells can provide an immune response faster and stronger against pathogenic antigens that invade again later.

When plasma cells secrete antibodies into the blood, the antibodies bind to antigens and cause antigen-antibody reactions to remove the antigens. One type of antibody specifically binds to only one type of antigen, and this specific binding is called the specificity of the antigen-antibody reaction. Antibodies adhere to invading microorganisms, thereby rendering the microorganisms immobile and preventing the microorganisms from penetrating body cells. In addition, the antibody-coated antigen triggers a chemical chain reaction with a complement, which is a series of proteins found in blood plasma. This complement reaction triggers lysis of the invading microorganism or attracts macrophages that eat the invading microorganisms. This type of antigen-antibody reaction and binding is called antigen-antibody reaction.

Humoral immunity can be divided into primary and secondary immune responses. In the primary immune response period, when an antigen first invades the body, lymphocytes differentiate to produce antibodies, and memory cells are formed at this time. The secondary immune response is a process in which, when the same antigen invades again, memory cells rapidly differentiate and proliferate into plasma cells and proliferate to produce a large amount of antibodies, thus triggering a faster and stronger immune response.

The adaptive immune system, composed of T and B lymphocytes, has powerful anticancer potential along with the ability to respond to a variety of tumor antigens. Additionally, the immune system has significant plasticity and memory components, and these attributes of the adaptive immune system make immunotherapy a more effective method among all cancer treatment options. However, although an endogenous immune response to cancer is observed in preclinical models and patients, this response is not effective, and established cancers are viewed as "self" and tolerated by the immune system. Due to this state of tolerance, tumors may exploit several distinct mechanisms to actively suppress anti-tumor immunity. These mechanisms include dysfunctional T-cell signaling, suppressive regulatory cells, and the co-opting of endogenous "immune checkpoints," which serve to down-modulate the intensity of adaptive immune responses and protect normal tissues from collateral damage caused by tumors to evade immune destruction.

The long-term battle between the human immune system and diseases, such as chronic infections and cancer, places a significant physiological burden on the host, and as a result, the immune response may reach a stalemate. T cells are exhausted and inactivated, giving viruses, bacteria or tumors an edge. Based on a number of molecular databases, researchers have identified nine types of exhausted T ("Tex") cells, which could have implications for fighting chronic infections, autoimmunity, and cancer. When normal T cells become exhausted, they develop defects in their germ- and tumor-fighting capabilities. Tex cells also express inhibitory receptor proteins on their surface that stall key biochemical pathways, provoke changes in control of gene expression, alter metabolism for making energy to fight infections and tumors, and prevent development of optimal immune function. Applying this type of assessment to exhausted T cells in the context of immunotherapy clinical trials might identify patients more likely to benefit from specific types of combination immunotherapies and may point to underlying mechanisms in the specific types of exhausted T cells responding to an infection or cancer.

New, highly effective immunotherapies that target the inhibitory receptors expressed by Tex cells, such as PD-1 or CTLA-4, can be breakthrough therapeutic methods for cancer treatment.

In this regard, until recently, cancer immunotherapy had focused substantial effort on approaches that enhance anti-tumor immune responses by adoptive-transfer of activated effector cells, immunization against relevant antigens, or providing non-specific immunostimulatory agents such as cytokines. In the past decade, however, intensive efforts to develop specific immune checkpoint pathway inhibitors have begun to provide new immunotherapeutic approaches for treating cancer, including the development of an antibody (Ab), ipilimumab (YERVOY®), that binds to and inhibits Cytotoxic T-Lymphocyte Antigen-4 (CTLA-4) for the treatment of patients with advanced melanoma.

Meanwhile, programmed death-1 (PD-1) is a key immune checkpoint receptor expressed by activated T and B cells and mediates immunosuppression. PD-1 is a 55-kDa type I transmembrane protein, which is part of the Ig gene superfamily and is well known as a co-inhibitory molecule on T cells. PD-1 is a member of the CD28 family of receptors, including CD28, CTLA-4, ICOS, PD-1, and BTLA, which are receptors expressed on activated B cells, T cells and myeloid cells. Two cell surface glycoprotein ligands for PD-1 have been identified, Programmed Death Ligand-1 (PD-L1) and Programmed Death Ligand-2 (PD-L2), that are expressed on antigen presenting cells as well as many human cancers and have been shown to downregulate T cell activation and cytokine secretion upon binding to PD-1 (Freeman et al., 2000; Latchman et al, 2001). It is known that PD-1 is not expressed in normal T cells in a normal state, but the expression thereof increases when activated. It has been reported that PD-L1 is expressed to some extent in normal T cells, and the expression thereof also increases when activated. PD-L1 is found in large quantities in many human cancers, and the interaction between PD-1 and PD-L1 transmits stimulating or inhibitory signals to T cells. In other words, due to the interaction between PD-1 and PD-L1, tumor-invasive lymphocytes decrease, T cell receptor-mediated proliferation decreases, and immune evasion by cancer cells occurs. Unlike CTLA-4, PD-1 primarily functions in peripheral tissues where activated T-cells may encounter the immunosuppressive PD-L1 (B7-H1) and PDL2 (B7-DC) ligands expressed by tumor and/or stromal cells [Flies et al., 2011; Topalian et al., 2012a]. When PD-L1 and PD-L2, proteins on the surface of cancer cells, bind to PD-1, a protein on the surface of T cells, T cells cannot attack cancer cells.

Intracellular signaling by PD-1 is essential to induce immune exhaustion of T cells in chronic infection. For example, it is known that PD-1 plays an important role in the process of chronicization of viral diseases including chronic hepatitis B and C. Therefore, inhibition of PD-1/PD-L1 interaction can induce antitumor activity.

Meanwhile, the specific antigen-antibody reaction system existing in the body is generally understood as independent immunity and is understood to be independent of other diseases, and thus has not been well used for the treatment of antigens or diseases until now. Rather, immunity formed against bacteria or viruses that infect the same organ interferes with the formation of antibodies against other infectious agents. As a method of overcoming this interference, a method of administering a chimeric antigen containing both the antigens of both infectious agents has been attempted, or an attempt has also been made to enhance the immune response to one antigen by administering a recombinant chimeric antigen containing both antigens. However, until now, there have been very few successful cases showing effective pharmacological activity by chimeric antigens.

Accordingly, the present inventors have made efforts to develop a method for effective treatment of cancer or infectious diseases, and as a result, have produced a chimeric antigen comprising: a specific foreign antigen domain capable of triggering a specific antigen-antibody reaction system in the body; and a peptide domain capable of binding to a disease-related target protein. In addition, the present inventors have found that, when the produced chimeric antigen is administered, the chimeric antigen can bind specifically to cancer or infected cells having a target protein on the cell membrane, while the other domain of the chimeric antigen can trigger an antigen-antibody reaction system that has already been formed in the body, thereby effectively removing the target cells and very effectively treating cancer or infectious diseases. Based on this finding, the present invention has been completed.

### [Disclosure]

### [Technical Problem]

Therefore, the present inventors have developed a novel therapeutic agent capable of maximizing the effect of immunotherapy by activating both the cellular immunity and humoral immunity or antibody-dependent cellular cytotoxicity of T cells and B cells using a chimeric antigen produced by fusion with an mRNA synthesized by *in vitro* transcription, that is, IVT mRNA.

Therefore, an object of the present invention is to provide a chimeric antigen for enhancing multiple immune functions wherein an immune response-inducing domain and a domain for inducing target cell-specific binding are fused to each other.

Another object of the present invention is to provide a pharmaceutical composition for preventing or treating cancer, the pharmaceutical composition containing, as an active ingredient, the chimeric antigen for enhancing multiple immune functions according to the present invention.

Still another object of the present invention is to provide a pharmaceutical composition for preventing or treating infectious disease, the pharmaceutical composition containing, as an active ingredient, the chimeric antigen for enhancing multiple immune functions according to the present invention.

Yet another object of the present invention is to provide a composition for enhancing immunity, the composition containing, as an active ingredient, the chimeric antigen for enhancing multiple immune functions according to the present invention.

Yet another object of the present invention is to provide a vaccine composition containing, as an active ingredient, the chimeric antigen for enhancing multiple immune functions according to the present invention.

### [Technical Solution]

To achieve the above objects, the present invention provides a chimeric antigen for enhancing multiple immune functions wherein an immune response-inducing domain and a domain for inducing target cell-specific binding are fused to each other.

In one embodiment of the present invention, the immune response-inducing domain may be a foreign viral antigen, and an antigen-antibody reaction may be formed by the antigen.

In one embodiment of the present invention, the immune response-inducing domain may be selected from the group consisting of hepatitis B virus antigen, hepatitis C virus antigen, hepatitis A virus antigen, AIDS virus antigen, MERS virus antigen, influenza A virus antigen, human papillomavirus antigen, lymphocytic choriomeningitis virus (LCMV) antigen, and herpes simplex virus (HSV) antigen.

In one embodiment of the present invention, the immune response-inducing domain may be hepatitis B virus core protein (HBc), hepatitis B virus e antigen (HBe), or hepatitis B virus surface protein (HBs).

In one embodiment of the present invention, the domain for inducing target cell-specific binding is a cell membrane protein capable of binding specifically to target cells, and may be selected from the group consisting of PD-1, PD-L1, PD-L2, CTLA-4, CD28, ICOS, OX40, OX40L, GITR, GITRL, CD40, CD40L, 4-1BB, 4-1BBL, B7-1, B7-2, B7-H1, B7-H2, B7-DC, CD80, CD160, BTLA, HVEM, DPP-4, NTCP, CD16, and Caveolin-1.

In one embodiment of the present invention, the immune response-inducing domain and the domain for inducing target cell-specific binding may be linked to each other by a linker peptide represented by SEQ ID NO: 19 or SEQ ID NO: 21 to increase the structural stability of the chimeric antigen.

In one embodiment of the present invention, the chimeric antigen for enhancing multiple immune functions may consist of a peptide sequence of SEQ ID NO: 9 or SEQ ID NO: 11.

The present invention also provides a pharmaceutical composition for preventing or treating cancer, the pharmaceutical composition containing, as an active ingredient, the chimeric antigen for enhancing multiple immune functions according to the present invention.

In one embodiment of the present invention, the cancer may be selected from the group consisting of lung cancer, stomach cancer, liver cancer, bone cancer, pancreatic cancer, skin cancer, head and neck cancer, skin melanoma, uterine cancer, ovarian cancer, rectal cancer, colorectal cancer, colon cancer, breast cancer, uterine sarcoma, fallopian tube carcinoma, endometrial carcinoma, cervical carcinoma, vaginal carcinoma, vulvar carcinoma, esophageal cancer, laryngeal cancer, small intestine cancer, thyroid cancer, parathyroid cancer, soft tissue sarcoma, urethral cancer, penile cancer, prostate cancer, chronic or acute leukemia, childhood solid tumor, differentiated lymphoma, bladder cancer, kidney cancer, renal cell carcinoma, renal pelvic carcinoma, primary central nervous system lymphoma, spinal cord tumor, brainstem glioma, and pituitary adenoma.

The present invention also provides a pharmaceutical composition for preventing or treating infectious disease, the pharmaceutical composition containing, as an active ingredient, the chimeric antigen for enhancing multiple immune functions according to the present invention.

In one embodiment of the present invention, the infectious disease may be hepatitis B virus, hepatitis C virus, hepatitis A virus, AIDS virus, MERS virus, influenza A virus, human papillomavirus, lymphocytic choriomeningitis virus (LCMV) or herpes simplex virus (HSV) infection.

The present invention also provides a composition for enhancing immunity, the composition containing, as an active ingredient, the chimeric antigen for enhancing multiple immune functions according to the present invention.

In one embodiment of the present invention, the chimeric antigen for enhancing multiple immune functions may: (i) bind specifically to diseased cells which are target cells; (ii) induce T cell activation by stimulating the restoration of exhausted CD8+ T cells; and (iii) enhance humoral immune response and activate the cytotoxicity of natural killer (NK) cells, by inducing an antigen-antibody reaction.

In one embodiment of the present invention, the inducing the antigen-antibody reaction in (iii) may include enhancing a specific antigen-antibody reaction, formed in the body of a subject, by the immune response-inducing domain of the chimeric antigen.

The present invention also provides a vaccine composition containing, as an active ingredient, the chimeric antigen for enhancing multiple immune functions according to the present invention.

### [Advantageous effects]

The present invention relates to a chimeric antigen, which binds specifically to target cells and enhances multiple immune functions, and the use thereof. The chimeric antigen for enhancing multiple immune functions includes an immune response-inducing domain and a domain for inducing target cell-specific binding, which are fused to each other. The chimeric antigen may bind specifically to diseased cells, induce T cell activation by enhancing the restoration of exhausted CD8+ T cells, and enhance humoral immune response and activate the cytotoxicity of natural killer (NK) cells, by inducing an antigen-antibody reaction. Thus, chimeric antigen may suppress immune evasion of diseased cells and enhance multiple immune functions, thereby more effectively preventing or treating cancer, infectious diseases and immune diseases.

### [Description of Drawings]

FIG. 1 is a schematic view showing a process in which a soluble chimeric antigen induces an antibody, formed in the body, to induce humoral immunity, and also showing that the chimeric antigen restores exhausted CD8+ T cells in target cancer or infected cells, and CD8+ T cells specific to the specific foreign antigen of the chimeric antigen are directed to target cancer or infected cells, so that reactivated T cells and cell-mediated cellular cytotoxicity act at the same time.
FIG. 2 shows the design of fusion genes, each encoding either a single antigen of HBcAg, HBeAg or HBsAg or a chimeric antigen of PD-1/HBc or PD-1/HBe, expected to have various molecular biological structures, in which a recombinant DNA fragment for producing an IVT mRNA encoding the single antigen or the chimeric antigen is designed to include an open reading frame, a T7 promoter, a 5' untranslated region (UTR), a 3' untranslated region (UTR), and the like.
FIG. 3 shows the results of producing DNA fragments encoding HBcAg, HBeAg, HBsAg and PD-1 single antigens and PD-1/HBc and PD-1/HBe chimeric antigens, respectively.
FIG. 4 shows the results of producing each IVT mRNA using, as a template, each of DNA fragments encoding HBcAg, HBeAg, HBsAg and PD-1 single antigens and PD-1/HBc and PD-1/HBe chimeric antigens.
FIG. 5 shows the results of an enzyme-linked immunosorbent assay performed to identify an antibody actually produced in animal 293T cells after inserting IVT mRNA encoding each of PD-1/HBc and PD-1/HBe chimeric antigens of the present invention into the cells.
FIG. 6 shows the results of Western blotting performed to identify an antibody actually produced in animal 293T cells after inserting IVT mRNA encoding each of PD-1/HBc and PD-1/HBe chimeric antigens of the present invention into the cells.
FIG. 7 shows the results of co-immunoprecipitation, which indicate that PD-1/HBc and PD-1/HBe chimeric antigens expressed in cells actually bound to a PD-L1 ligand in the cells.
FIG. 8 shows the results of measuring particle size using a nanoparticle analyzer (Zetasizer) after encapsulating the single antigen and chimeric antigen IVT mRNAs, synthesized in one example of the present invention, into LNPs.
FIG. 9 shows the results of measuring the efficiency with which mRNA was actually encapsulated into particles, after the single antigen and chimeric antigen IVT mRNAs synthesized in one example of the present invention are encapsulated into LNPs.
FIG. 10 shows the results of enzyme-linked immunosorbent assay, which indicate that chimeric antigen proteins were actually produced in animal 293T cells after LNPs encapsulated with IVT mRNA are inserted into the cells.
FIG. 11 shows the results of enzyme-linked immunosorbent assay, which indicate that antigen proteins were actually produced from chimeric antigen PD-1/HBe IVT mRNAs after LNPs encapsulated with the IVT mRNAs are injected into C57BL/6 mice.
FIG. 12 shows the results of enzyme-linked immunosorbent assay, which indicate that an antibody against each antigen was actually produced in C57BL/6 mice inoculated with each of the single antigens HBcAg and HBeAg proteins before the mice were treated with chimeric antigens.
FIG. 13 shows the results obtained by injecting HBcAg and HBeAg proteins into mice to form antibodies against these proteins, transplanting mouse melanoma B16F10 cancer cells into the mice, injecting, into the mice, LNPs encapsulated with IVT mRNAs encoding PD-1/HBc and PD-1/HBe, respectively, and confirming that growth of the cancer tissue was inhibited by the chimeric antigens produced in the bodies of the mice.
FIG. 14 shows the results obtained by forming antibodies against HBcAg and HBeAg in C57BL/6 mice, transplanting melanoma cancer B16F10 cells into the mice to induce the production of cancer tissue, injecting, into the mice, LNPs encapsulated with IVT mRNAs encoding PD-1/HBc and PD-1/HBe, respectively, and confirming that the survival rate of the mice was increased by the chimeric antigens produced in the bodies of the mice.
FIG. 15 shows the results obtained by forming antibodies against HBcAg and HBeAg in C57BL/6 mice, transplanting melanoma cancer B16F10 cells into the mice to induce the production of cancer tissue, injecting, into the mice, LNPs encapsulated with IVT mRNAs encoding PD-1/HBc and PD-1/HBe, respectively, and confirming that the survival rate of the mice increased after the LNPs were injected into the mice (treatment with the chimeric antigens) compared before injection of the LNPs.
FIG. 16 shows the results obtained by forming an antibody against an HBcAg antigen in C57BL/6 mice, transplanting melanoma cancer B16F10 cells into the mice to induce the production of cancer tissue, injecting, into the mice, LNPs encapsulated with an IVT mRNA encoding PD-1/HBc, and examining the relationship between the relative amount of the antibody to the HBcAg antigen and the size of the cancer tissue before and after injection of the LNPs.

### [Best Mode]

The present invention is characterized by providing a chimeric antigen for enhancing multiple immune functions. Specifically, the present invention is characterized by providing a chimeric antigen for enhancing multiple immune functions wherein an immune response-inducing domain and a domain for inducing target cell-specific binding are fused to each other.

In particular, the chimeric antigen for enhancing multiple immune functions, which is provided according to the present invention, is produced by fusing a domain, which is capable of binding specifically to a disease-related target protein, to a specific foreign antigen domain capable of triggering a specific antigen-antibody reaction system formed in the body. When the chimeric antigen of the present invention is administered to a subject, the chimeric antigen may bind specifically to specific diseased cells (e.g., cancer cells or infected cells) having a target protein on the cell membrane, and the other domain of the chimeric antigen, that is, the immune response-inducing domain (specific foreign antigen domain), may trigger an antigen-antibody reaction system already formed in the body. Thus, the chimeric antigen of the present invention was designed such that it can efficiently remove target diseased cells.

Thus, the use of the chimeric antigen of the present invention may very efficiently treat a target disease by reactivating the immune system (immune response) by a specific antigen-antibody reaction system already formed in the subject.

Looking at the components of the chimeric antigen for enhancing multiple immune functions according to the present invention, the chimeric antibody is largely composed of a fusion protein in which an immune response-inducing domain and a domain for inducing target cell-specific binding are fused to each other.

The immune response-inducing domain may be a specific foreign antigen domain which is a foreign viral antigen, and an antigen-antibody reaction in a subject, to which the chimeric antigen has been administered, may be stably formed by the immune response-inducing domain.

The immune response-inducing domain that may be included in the chimeric antigen of the present invention may include a foreign antigen which is a protein, glycoprotein, coat protein, core protein or functional protein derived from viruses, bacteria or organisms associated with various infectious diseases. Preferably, the immune response-inducing domain may be selected from the group consisting of, but not limited to, hepatitis B virus antigen, hepatitis C virus antigen, hepatitis A virus antigen, AIDS virus antigen, MERS virus antigen, influenza A virus antigen, human papillomavirus antigen, lymphocytic choriomeningitis virus (LCMV) antigen, and herpes simplex virus (HSV) antigen. More preferably, the immune response-inducing domain may be hepatitis B virus core protein (HBc), hepatitis B virus e antigen (HBe), or hepatitis B virus surface protein (HBs).

The hepatitis B virus core protein antigen (HBcAg) may consist of the peptide sequence of SEQ ID NO: 1, the hepatitis B virus antigen (HBeAg) may consist of the peptide sequence of SEQ ID NO: 3, and the hepatitis B virus surface protein antigen (HBsAg) may consist of the peptide sequence of SEQ ID NO: 5.

In addition, the polynucleotide encoding the peptide of SEQ ID NO: 1 is represented by SEQ ID NO: 2, the polynucleotide encoding the peptide of SEQ ID NO: 3 is represented by SEQ ID NO: 4, and the polynucleotide encoding the peptide of SEQ ID NO: 5 is represented by SEQ ID NO: 6.

In addition, the domain for inducing target cell-specific binding may be a protein mainly related to normal life activity in the body, specifically a soluble protein or cell membrane protein capable of physically binding to a receptor or a ligand on the cell membrane. The domain for inducing target cell-specific binding may be selected from the group of, but not limited to, PD-1, PD-L1, PD-L2, CTLA-4, CD28, ICOS, OX40, OX40L, GITR, GITRL, CD40, CD40L, 4-1BB, 4-1BBL, B7-1, B7-2, B7-H1, B7-H2, B7-DC, CD80, CD160, BTLA, HVEM, DPP-4, NTCP, CD16 and Caveolin-1.

In one example of the present invention, the cell membrane protein PD-1 capable of binding specifically to target cells was used as the domain for inducing target cell-specific binding. Cell membrane proteins play a key role in the immune checkpoint system, and it is known that PD-1 attached to the cell membrane is easily secreted extracellularly (Cellular Immunology, 2005, 235:109). Accordingly, in the present invention, PD-1 was converted into a protein that firmly binds to its receptor, PD-L1, and thus a recombinant PD-1 protein was used, which may be secreted extracellularly and has a high binding affinity for its receptor. The peptide sequence of the PD-1 protein used in the present invention is represented by SEQ ID NO: 7, and the polynucleotide sequence encoding the polypeptide of SEQ ID NO: 7 is represented by SEQ ID NO: 8.

In addition, the chimeric antigen of the present invention was designed such that the immune response-inducing domain and the domain for inducing target cell-specific binding are linked to each other by a linker consisting of a specific sequence in order to maximize the structural stability of the chimeric antigen and the immune response thereto. It was confirmed that there is a difference in the structural stability of the chimeric antigen depending on the sequence of the linker peptide, and based on this fact, a linker peptide effective to increase the stability of the chimeric antigen and the immune response thereto was selected. Linker peptide 1 that may be used in the present invention is represented by SEQ ID NO: 19 and consists of 46 amino acids, and the polynucleotide sequence encoding linker peptide 1 is represented by SEQ ID NO: 20. In addition, linker peptide 2 that may be used in the present invention is represented by SEQ ID NO: 21 and consists of 24 amino acids, and the polynucleotide sequence encoding linker peptide 2 is represented by SEQ ID NO: 22.

In one example of the present invention, as chimeric antigens for enhancing multiple immune functions, PD-1/HBc and PD-1/HBe, which are soluble chimeric antigens, were produced. These chimeric antigens were produced using hepatitis B virus core protein (HBcAg) and hepatitis B virus e antigen protein (HBeAg), respectively, as an immune response-inducing domain, and using soluble PD-1, which has binding affinity for PD-L1 and is extracellularly secreted, as a domain for inducing target cell-specific binding. In addition, in one example of the present invention, the PD-1 and HBc domains were linked to each other by linker peptide 1 of SEQ ID NO: 19, and the PD-1 and HBe domains were linked to each other by linker peptide 2 of SEQ ID NO: 21.

In addition, HBcAg and HBeAg single antigens and PD-1/HBc and PD-1/HBe chimeric antigens were designed to contain most of the amino acid sequences that easily induce antibody formation, and each of the antigen proteins was designed to contain a signal peptide at the amino terminus thereof so that each antigen protein is easily secreted extracellularly after intracellular production thereof. The signal peptide was synthesized according to a method well known to those skilled in the art (PLoS One. 2016.19:11; Mol Ther. 2005, 11(3):435; Journal of Biotechnology, 2007, 128(4):705; Trends in Cell and Molecular Biology, Improving mammalian cell factories: The selection of signal peptide has a major impact on recombinant protein synthesis and secretion in mammalian cells). In the present invention, for a number of signal peptides synthesized, each of various signal peptide sequences was linked to the amino terminus of each of the antigen proteins, and whether the produced antigens were easily secreted extracellularly was verified. Then, signal peptides included in the antigens that were easily secreted extracellularly were selected.

In the present invention, in order to facilitate extracellular secretion of the chimeric antigen of the present invention through the experiment, signal peptide 1, represented by SEQ ID NO: 23 and consisting of 21 amino acids, or signal peptide 2, represented by SEQ ID NO: 25 and consisting of 17 amino acids, was used. Here, the polynucleotide sequence encoding the polypeptide of SEQ ID NO: 23 is represented by SEQ ID NO: 24, and the polynucleotide sequence encoding the polypeptide of SEQ ID NO: 25 is represented by SEQ ID NO: 26.

As described above, the chimeric antigen for enhancing multiple immune functions according to the present invention has a structure in which the immune response-inducing domain and the domain for inducing target cell-specific binding are fused to each other. In one example of the present invention, as the chimeric antigen for enhancing multiple immune functions, each of PD-1/HBcAg and PD-1/HBeAg chimeric antigens was produced.

The peptide sequence for the PD-1/HBcAg chimeric antigen produced in the present invention is represented by SEQ ID NO: 9, and the polynucleotide sequence encoding the same is represented by SEQ ID NO: 10.

The polypeptide of SEQ ID NO: 9 representing the PD-1/HBcAg chimeric antigen of the present invention contains the sequence of signal peptide 1 for extracellular secretion, and the sequence of signal peptide 1 consists of a total of 21 amino acids (amino acids 1 to 21 in SEQ ID NO: 9).

In addition, the polypeptide of SEQ ID NO: 9 representing the PD-1/HBcAg chimeric antigen of the present invention contains the sequence of linker peptide 1, and the sequence of linker peptide 1 consists of a total of 46 amino acids (amino acids 232 to 277 in SEQ ID NO: 9).

In addition, the polynucleotide sequence encoding the polypeptide for the PD-1/HBcAg chimeric antigen of SEQ ID NO: 9 is represented by SEQ ID NO: 10. Here, the sequence of the polynucleotide encoding signal peptide 1 in SEQ ID NO: 10 consists of a total of 63 nucleotides (nucleotides 1 to 63 in SEQ ID NO: 10), and the sequence of the polynucleotide encoding linker peptide 1 in SEQ ID NO: 10 consists of nucleotides 694 to 831 in SEQ ID NO: 10.

In addition, the peptide sequence of the PD-1/HBeAg chimeric antigen of the present invention is represented by SEQ ID NO: 11, and the polynucleotide sequence encoding the same is represented by SEQ ID NO: 12.

The polypeptide of SEQ ID NO: 11 representing the PD-1/HBeAg chimeric antigen of the present invention contains the sequence of signal peptide 2 for extracellular secretion, and the sequence of signal peptide 2 consists of a total of 17 amino acids (amino acids 1 to 17 in SEQ ID NO: 11).

In addition, the polypeptide of SEQ ID NO: 9 representing the PD-1/HBeAg chimeric antigen of the present invention contains the sequence of linker peptide 2, and the sequence of linker peptide 2 consists of a total of 24 amino acids (amino acids 228 to 251 in SEQ ID NO: 11).

In addition, the polynucleotide sequence encoding the polypeptide for the PD-1/HBeAg chimeric antigen of SEQ ID NO: 11 is represented by SEQ ID NO: 12. Here, the sequence of the polynucleotide encoding signal peptide 2 in SEQ ID NO: 12 consists of a total of 72 nucleotides (nucleotides 1 to 51 in SEQ ID NO: 12), and the sequence of the polynucleotide encoding linker peptide 2 in SEQ ID NO: 12 consists of nucleotides 682 to 753 in SEQ ID NO: 12.

Meanwhile, the chimeric antigen for enhancing multiple immune functions according to the present invention may be used to prevent or treat cancer or infectious disease by activating the immune response.

The chimeric antigen of the present invention contains the domain for inducing target cell-specific binding, and thus may bind specifically to diseased cells, which are target cells, and the chimeric antigen contains the immune response-inducing domain, and thus may induce T cell activation by stimulating the restoration of exhausted CD8+ T cells, and at the same time, enhance humoral immune response and the cytotoxicity of natural killer (NK) cells by inducing an antigen-antibody reaction. Therefore, the chimeric antigen of the present invention may enhance multiple immune functions.

In particular, the enhancement of multiple immune functions by the immune response-inducing domain means treating a subject, who is exposed to a foreign antigen and in whom a specific and stable antigen-antibody reaction system for the foreign antigen, with the chimeric antigen of the present invention that contains the foreign antigen, thereby reactivating the antigen-antibody reaction, thus activating both humoral immunity and cellular immunity at the same time.

Therefore, the present invention may provide a pharmaceutical composition for preventing or treating cancer, the pharmaceutical composition containing, as an active ingredient, the chimeric antigen for enhancing multiple immune functions according to the present invention.

Examples of the cancer include, but are not limited to, lung cancer, stomach cancer, liver cancer, bone cancer, pancreatic cancer, skin cancer, head and neck cancer, skin melanoma, uterine cancer, ovarian cancer, rectal cancer, colorectal cancer, colon cancer, breast cancer, uterine sarcoma, fallopian tube carcinoma, endometrial carcinoma, cervical carcinoma, vaginal carcinoma, vulvar carcinoma, esophageal cancer, laryngeal cancer, small intestine cancer, thyroid cancer, parathyroid cancer, soft tissue sarcoma, urethral cancer, penile cancer, prostate cancer, chronic or acute leukemia, childhood solid tumor, differentiated lymphoma, bladder cancer, kidney cancer, renal cell carcinoma, renal pelvic carcinoma, primary central nervous system lymphoma, spinal cord tumor, brainstem glioma, and pituitary adenoma.

The present invention may also provide a pharmaceutical composition for preventing or treating infectious disease, the pharmaceutical composition containing, as an active ingredient, the chimeric antigen for enhancing multiple immune functions according to the present invention.

In the present invention, the term "infectious disease" refers to a disease in which various infectious agents or components thereof, including foreign viruses, bacteria and protozoa cause, mediate, or otherwise contribute to a pathological condition in mammals. Examples of the infectious disease include, but are not limited to, hepatitis B virus, hepatitis C virus, hepatitis A virus, AIDS virus, MERS virus, influenza A virus, human papillomavirus, lymphocytic choriomeningitis virus (LCMV) or herpes simplex virus (HSV) infection.

The present invention may also provide a composition for enhancing immunity, the composition containing, as an active ingredient, the chimeric antigen for enhancing multiple immune functions according to the present invention. This composition for enhancing immunity may be used as a pharmaceutical composition for the prevention or treatment of an immune disease caused by abnormalities in immune function.

In the present invention, the term "immune disease" refers to a disease in which components of the mammalian immune system cause, mediate, or otherwise contribute to a pathological condition in a mammal. In addition, the "immune disease" may include all diseases in which stimulation or interruption of immune response has a compensatory effect on the progression of the disease.

In one example of the present invention, a PD-1/HBc chimeric antigen, in which HBcAg and PD-1 are fused to each other, and a PD-1/HBe chimeric antigen in which HBeAg and PD-1 are fused to each other, were produced as mRNAs (IVT mRNAs; *in vitro* transcription mRNAs), respectively, through *in vitro* transcription, and each of the produced PD-1/HBc mRNA and PD-1/HBe mRNA was introduced to diseased cells, and then analysis was made as to whether each of the chimeric antigens bound selectively to the PD-L1 ligand. As a result, it could be confirmed that the PD-1/HBc and PD-1/HBe chimeric antigens all bound to the PD-L1 ligand.

In addition, in the present invention, each of the HBcAg and HBeAg proteins was injected into mice to produce anti-HBcAg and anti-HBeAg antibodies capable of binding to the chimeric antigens PD-1/HBc and PD-1/HBe, respectively, and then analysis was conducted to determine whether the antibodies were produced. As a result, it was confirmed that specific antibodies against HBcAg and HBeAg were produced.

Based on these results, the present inventors have found that, when the chimeric antigen of the present invention is administered to a subject, the chimeric antigen of the present invention may bind to a diseased cell-specific target area, and at the same time, bind to an antibody present in the subject, thereby promoting humoral immunity, and as a result, effectively prevent or treat the target disease by removing diseased cells through immune enhancement activity.

Furthermore, in order to evaluate the anticancer activity of the chimeric antigen of the present invention through an animal experiment, each of HBsAg and HBcAg were inoculated into mice to form an antibody against each of the antigens, and mouse cancer B16F10 cells having PD-L1 on the cell membrane were transformed into the mice to produce cancer tissue, and then each of the chimeric antigens of the present invention was injected into the mice. As a result, it could be seen that the group injected with each of the single antigens HBcAg and HBeAg alone showed little effect on cancer cell death, whereas, in the group injected with each of the chimeric antigens PD-1/HBc mRNA and PD-1/HBe mRNA of the present invention, growth of the cancer tissue was effectively inhibited and, at the same time, the survival rate of the mice significantly increased.

Through the above-described results, it could be confirmed that the chimeric antigen for enhancing multiple immune functions according to the present invention has the effect of preventing and treating cancer or infectious disease. In particular, it could be confirmed that the chimeric antigen of the present invention may induce recovery from T cell exhaustion caused by cancer or infected cells by blocking the signaling pathway of PD-1 that mediates immune inhibition and blocking of the binding of PD-1 to PD-L1, and may enhance immune response by activating the ability of T cells to secrete cytokines.

That is, it could be seen that the chimeric antigen of the present invention can promote the activation of target-specific CD8 + T cells, which have been already formed in the body but disabled by T cell exhaustion. In addition, natural killer (NK) cells, macrophages and monocytes can more rapidly and strongly prevent or treat diseases, which are caused by the interaction between PD-1 and PD-L1, than general therapeutic antibodies alone, through antibody-dependent cellular cytotoxicity thereof. The diseases caused by the interaction between PD-1 and PD-L1 may be the above-described infectious diseases.

In the present invention, the pharmaceutical composition may contain one or more pharmaceutically acceptable carriers, excipients or diluents, in addition to a pharmaceutically effective amount of the chimeric antibody of the present invention. As used herein, the pharmaceutically effective amount refers to an amount sufficient to prevent, ameliorate and treat symptoms of cancer or infectious disease.

The chimeric antigen according to the present invention may be administered at a dose of 0.1 pg to 1 g per kg patient body weight, and the pharmaceutically effective amount may be appropriately changed according to the severity of disease symptoms, the patient's age, body weight, health condition and sex, the route of administration, and the duration of treatment.

In addition, the term "pharmaceutically acceptable" as used herein refers to a composition which is physiologically acceptable and, when administered to human beings, generally does not cause allergic reactions, such as gastrointestinal disorder and dizziness, or similar reactions thereto. Examples of the carrier, excipient and diluent include lactose, dextrose, sucrose, sorbitol, mannitol, xylitol, erythritol, maltitol, starch, gum acacia, alginate, gelatin, calcium phosphate, calcium silicate, cellulose, methyl cellulose, polyvinyl pyrrolidone, water, methyl hydroxybenzoate, propyl hydroxybenzoate, talc, magnesium stearate, and mineral oil. In addition, the composition may further contain a filler, an anti-aggregating agent, a lubricant, a wetting agent, a fragrance, an emulsifying agent and a preservative.

The composition of the present invention may be formulated so as to provide rapid, sustained, or delayed release of the active ingredients after administration to the patient by employing procedures well known in the art. The formulation may be provided in the form of a powder, granule, tablet, emulsion, syrup, aerosol, soft or hard gelatin capsule, sterile injectable solution, or sterile powder.

In addition, the composition for preventing or treating cancer or infectious disease according to the present invention may be administered through various routes, including oral, transdermal, subcutaneous, intravenous and intramuscular routes. The dose of the active ingredient may be appropriately selected according to various factors such as the route of administration, the patient's age, sex, body weight, and disease severity. The composition for preventing or treating cancer or infectious disease according to the present invention co-administered with a known compound having the effect of preventing, ameliorating or treating symptoms of cancer or infectious disease.

Furthermore, the present invention may provide a method for treating cancer or infectious disease, the method including a step of administering the chimeric antigen of the present invention to a mammal with cancer or infectious disease other than humans.

In addition, suitable routes of administration include oral, intranasal or transmucosal administration; as well as parenteral delivery, including intra-abdominal, intrathecal, intraventricular, intraperitoneal, intraocular, intramuscular, subcutaneous, intravenous or intramedullary injection.

Furthermore, the present invention may provide a vaccine composition against cancer or infectious disease, the vaccine composition containing, as an active ingredient, the chimeric antigen for enhancing multiple immune functions according to the present invention.

Although the vaccine composition of the present invention may be administered alone, it is preferably co-administered with an adjuvant. The adjuvant is a substance that non-specifically promotes the immune response to the antigen in the initial process of activation of immune cells, and refers to an agent or molecule that enhances immunity by increasing the activity of immune system cells, although not an immunogen for a host (Annu. Rev. Immunol, 1986, 4:369) . Adjuvants that may be administered together with the vaccine composition of the present invention to enhance the immune response include any of a variety of adjuvants, and known adjuvants typically include Freund adjuvant, aluminum compounds, muramyl dipeptide, lipopolysaccharide (LPS), monophosphoryl lipid A, and Quil A. The adjuvant may be administered simultaneously with the vaccine composition or may be administered sequentially at time intervals.

In addition, the vaccine composition of the present invention may further contain a solvent, an excipient, and the like. Examples of the solvent include, but are not limited to, physiological saline, and distilled water, and examples of the excipient include, but are not limited to, aluminum phosphate, aluminum hydroxide, and aluminum potassium sulfate. The vaccine composition may further contain a substance that is commonly used in the manufacture of vaccines in the art to which the present invention pertains.

The vaccine composition of the present invention may be prepared by a method that is commonly used in the art to which the present invention pertains. The vaccine composition of the present invention may be prepared into an oral or parenteral formulation, and is preferably prepared as an injectable solution which is a parenteral formulation. The vaccine composition may be administered by an intradermal, intramuscular, intraperitoneal, intravenous, subcutaneous, intranasal or epidural route.

The vaccine composition of the present invention can be administered in an immunologically effective amount to a subject. The "immunologically effective amount" refers to an amount sufficient to exhibit a disease preventing effect and an amount that does not cause side effects or serious or excessive immune response. The exact dose of the vaccine composition varies depending on the specific immunogen to be administered, and may be easily determined by a person skilled in the art depending on factors well known in the field of medicine, including the subject's age, weight, health, sex, and sensitivity to the drug, the route of administration, and the mode of administration. The vaccine composition may be administered once or several times.

### [Mode for Invention]

Hereinafter, the present invention will be described in more detail with reference to examples. These examples are provided to explain the present invention in more detail, and the scope of the present invention is not limited to these examples.

### <Example 1>

### Production of Fusion Gene Encoding HBc, HBe, HBs, PD-1/HBc or PD-1/HBe Chimeric Antigen

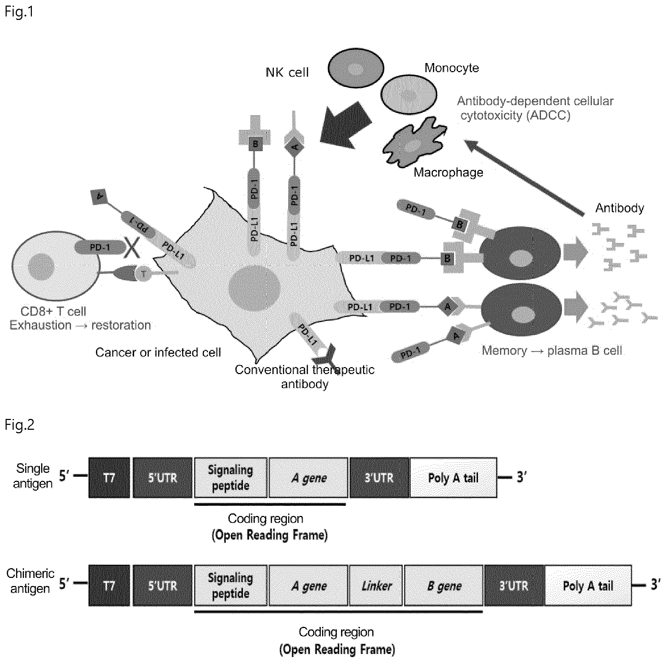

FIG. 2 shows a schematic view of a DNA fragment for producing an IVT mRNA (*in vitro* transcribed mRNA) by *in vitro* transcription of each gene encoding each of hepatitis B core antigen protein (HBcAg), hepatitis B e antigen protein (HBeAg), hepatitis B surface antigen protein (HBsAg), PD-1, and soluble chimeric antigens PD-1/HBc and PD-1/HBe. As shown in FIG. 2, the DNA fragment for producing the IVT mRNA was constructed with reference to an mRNAExpress mRNA Synthesis kit (System Biosciences, USA) so as to contain an open reading frame, a T7 promoter nucleotide sequence, a 5' untranslated nucleotide sequence (5'UTR) and a 3' untranslated nucleotide sequence (3'UTR). The DNA fragment was designed such that the open reading frame (i.e., the target gene) was linked and inserted in a cassette form. At this time, the order of the open reading frames encoding the immune response-inducing domain of the chimeric antigen and the target cell-specific binding inducing domain thereof is not fixed, but the positions of both the inducing domains may also be interchanged. That is, in FIG. 2, the immune response-inducing domain and the target cell-specific binding inducing domain of the chimeric antigen are positioned at "A gene" and "B gene" linked to each other by a linker, and the positions thereof may be interchanged.

In addition, the DNA nucleotide sequences of IVT mRNAs (*in vitro* transcribed mRNAs) for hepatitis B core antigen (HBcAg), hepatitis B e antigen (HBeAg), hepatitis B surface antigen (HBsAg), PD-1, and soluble chimeric antigens PD-1/HBcAg and PD-1/HBeAg are represented by SEQ ID Nos: 13 (HBcAg), 14 (HBeAg), 15 (HBsAg), 16 (PD-1), 17 (PD-1/HBcAg) and 18 (PD-1/HBeAg), respectively.

In addition, the nucleotide sequence of SEQ ID NO: 17 is the nucleotide sequence of a DNA template encoding PD-1/HBcAg IVT mRNA, and contains not only the coding sequence of PD-1/HBcAg, but also the nucleotide sequences of the T7 promoter, the 5'UTR and the 3'UTR.

In addition, the nucleotide sequence of SEQ ID NO: 18 is the nucleotide sequence of a DNA template encoding PD-1/HBeAg IVT mRNA, and contains not only the coding sequence of PD-1/HBeAg, but also the nucleotide sequences of the T7 promoter, the 5'UTR and the 3'UTR.

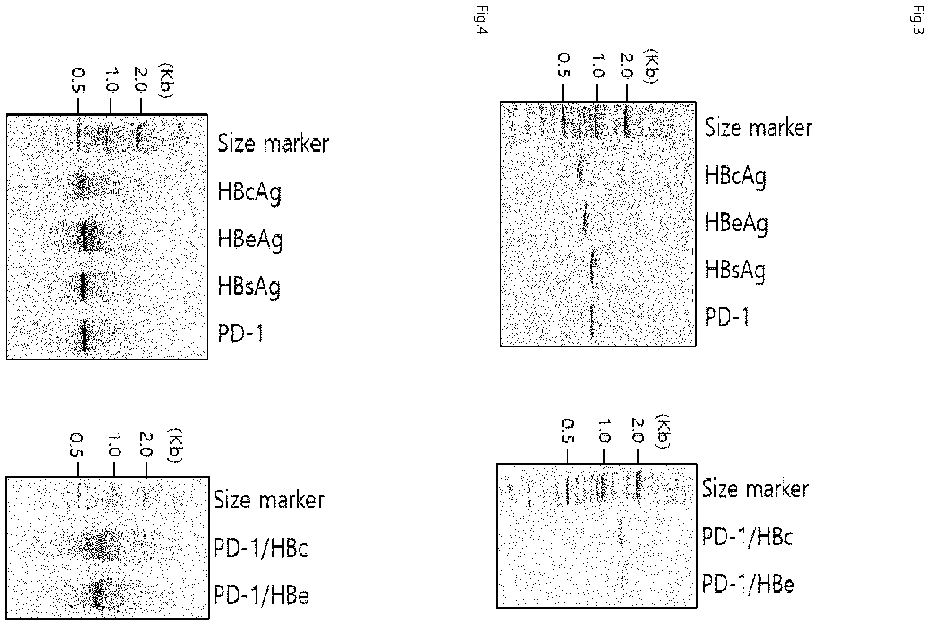

After completion of transcription, a series of adjacent "polyadenylation(poly(A) tails" was added to the 3' end of the RNA molecule by poly A polymerase, and this process was designed such that a poly(A) sequence consisting of at least 50 adenines was included at the design time by a method well known to those skilled in the art. The designed DNA fragments were synthesized by IDT (USA). Using, as a template, 10 ng of a vector DNA containing each antigen gene constructed by IDT, gene DNA amplification was performed using the polymerase chain reaction (PCR) primers of the following SEQ ID NOs: 27 and 28. Each PCR reaction product was purified by an elution method well known to those skilled in the art, and then used as a template for an *in vitro* transcription reaction (FIG. 3).

Primer nucleotide sequence (primer 1) of SEQ ID NO 27:
5'-AGATCTTAATACGACTCACTATAGGGAAATAAGA-3'

Primer nucleotide sequence (primer 2) of SEQ ID NO 28:

5′-TTTTTTTTTT TTTTTTTTTT TTTTTTTTTT TTCTTTTTTT
TTTTTTTTTT TTTTTTTTTT TTTTTAATAT TCTTCCTACT CAGGCTTTAT
TCAAAGACC-3′

### <Example 2>

### Synthesis of IVT mRNA from DNA Fragment Encoding Single Antigen or Chimeric Antigen

Artificial mRNA was synthesized by in vitro transcription using the template DNA produced in Example 1, and this synthesis was performed using the HiScribe T7 ARCA mRNA kit with tailing (New England Biolabs, USA), an mRNA production kit. Specifically, 1 µg of each purified DNA fragment containing the nucleotide sequence of the single or chimeric antigen gene of each of HBcAg, HBeAg, HBsAg, PD-1, PD-1/HBc and PD-1/HBe, 10 µl of 2x ARCA/NTP mix and 2 µl T7 RNA polymerase mix were mixed, and distilled water was added to the mixture to form 20 µl of a reaction mixture which was then allowed to react at 37°C for 60 minutes. After completion of the reaction, 2 µl DNase was added thereto, and then the reaction mixture was left to stand at 37°C for 15 minutes to completely remove residual DNA. The IVT mRNA obtained from the reaction was purified using an RNA purification kit well known to those skilled in the art, and then mRNA production was confirmed by 1.2% agarose gel electrophoresis using 0.5x TAE buffer (FIG. 4).

The polypeptide sequence of the single antigen protein HBcAg expressed from the single antigen mRNA produced through the above-described process is represented by SEQ ID NO: 1, the polypeptide sequence of the single antigen protein HBeAg is represented by SEQ ID NO: 3, the polypeptide sequence of the single antigen protein HBsAg is represented by SEQ ID NO: 5, and the polypeptide sequence of PD-1 is represented by SEQ ID NO: 7. In addition, the polypeptide (amino acid) sequence of the chimeric antigen protein PD-1/HBc expressed from the other chimeric antigen mRNA (SEQ ID NO: 17) is represented by SEQ ID NO: 9, and the polypeptide (amino acid) sequence of the chimeric antigen protein PD-1/HBe expressed from the other chimeric antigen mRNA (SEQ ID NO: 18) is represented by SEQ ID NO: 11.

### <Example 3>

### Analysis of Expression of Chimeric Antigen-Encoding IVT mRNA in Human 293T Cells

Each of the IVT mRNAs purified in Example 2 was transfected into the human cell line HEK293T (ATCC, CRL-3216). The HEK293T cell line is a cancerous kidney cell line originating from the human embryonic kidney. The cells were cultured for 3 days with MEM medium (GIBCO, #11095-080) (supplemented with 10% FBS (Fetus Bovine Serum: GIBCO, #16000-028) and 1% ABAM (GIBCO BRL, #15240-013)) in a T75 flask. After culture, the cells were washed with 1x PBS buffer, and then detached from the flask bottom by treatment with trypsin. Thereafter, the isolated cells were centrifuged, the supernatant was discarded, and the cells were re-suspended in 1x PBS buffer and then seeded again at a density of 5 x 10⁵ cells/dish. After 24 hours, it was confirmed that the cells are well attached to the surface of the culture dish. Then, the cells were transfected with each of the chimeric antigen-encoding IVT mRNAs (PD-1/HBc and PD-1/HBe) using Lipofectamine Messenger Max Transfection Reagent (Invitrogen, #LMRNA003), an mRNA transfection reagent, according to a method known to those skilled in the art. The transfected cells were dispersed in a 60-mm culture dish and incubated in a 5% CO₂ cell incubator for 2 days.

After 24 hours of incubation, the cell culture medium was harvested, and after 48 hours, the attached cells and the cell culture medium were harvested in a 1.5-ml tube. In order to examine whether the produced and secreted single or chimeric antigen was present in the harvested cells and cell culture medium, enzyme-linked immunosorbent assay (ELISA) was performed using a PD-1 ELISA kit (Arigo Biolaboratories, #ARG81342).

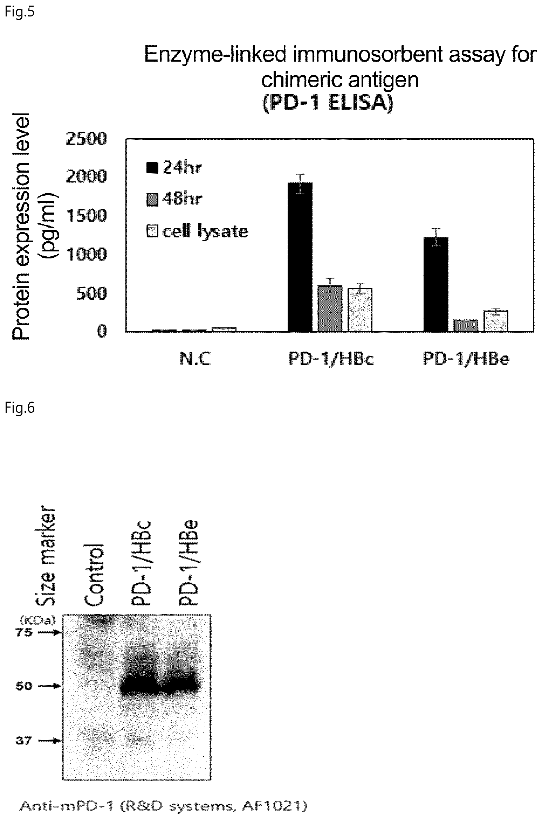

As a result, as shown in FIG. 5, it could be confirmed that the PD-1 protein for the IVT-mRNA PD-1 produced according to the method of the present invention was detected in both the culture medium and the cells after transfection into the cell line. Thereby, the present inventors could confirm that all the desired chimeric antigens were sufficiently secreted extracellularly. In addition, protein was extracted from the harvested cells, subjected to SDS-PAGE electrophoresis, and then subjected to Western blotting using anti-PD-1 antibody (R & D System, AF1021). As a result, as shown in FIG. 6, it could be confirmed that the desired chimeric antigens were produced.

In addition, the extract from the harvested cells was also used as a sample for immunoprecipitation which can show binding to the PD-L1 protein present on the cell membrane.

### <Example 4>

### Immunoprecipitation for Analysis of Binding between PD-L1 Protein Present on Cell Membrane and Chimeric Antigen Expressed in 293T

Whether the single antigen (PD-1) and chimeric antigen (PD-1/HBc or HBsAg/PD-1) produced in the present invention bind to PD-L1 present on the cell membrane was analyzed by immunoprecipitation (IP). To this end, an antibody against PD-L1 was added to obtain a precipitate of each of PD-L1/PD-1/anti-PD-L1 antibody, PD-L1/PD-1/HBc/anti-PD-L1 antibody and PD-L1/HBsAg/PD-1/anti-PD-L1 antibody, and the precipitate was electrophoresed, and then the interaction of PD-L1 with PD-1, PD-1/HBc or HBsAg/PD-1 was analyzed by co-immunoprecipitation (co-IP).

Specifically, cancer cells having PD-L1 on the cell membrane were transfected with the IVT mRNA encoding the PD-1, PD-1/HBc or PD-1/HBe protein using electroporation or Lipofectamine Messenger Max Transfection Reagent (Invitrogen, #LMRNA003) mRNA transfection reagent, and after 2 days, the cells were harvested and lysed with RIPA buffer. After the cell debris was removed by centrifugation, the supernatant was transferred into a 1.5-ml test tube, and a suitable amount of an anti-PD-Ll antibody or a specific marker protein antibody was added thereto and mixed therewith, and then incubated for 1 hour to form an antigen-antibody complex (immune complex). Next, the complex was added to immobilized Protein A or Protein G agarose gel, and then incubated with rotation for 2 hours so as to adhere to the gel. Next, unbound proteins were removed from the precipitated complex by washing several times, and the precipitated complex was subjected to SDS-PAGE electrophoresis, and then subjected to Western blotting using each antigen-specific antigen.

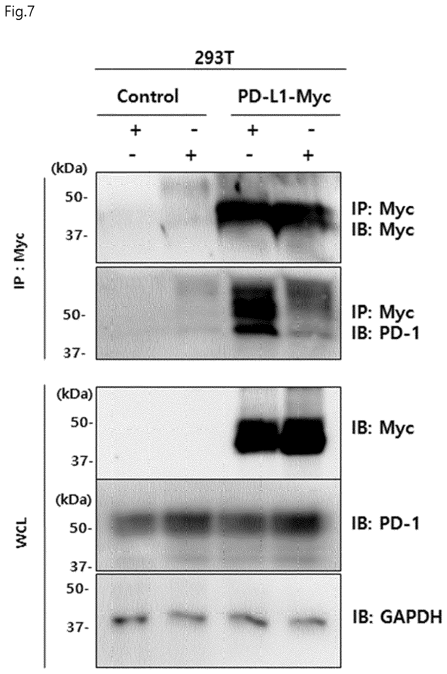

As a result, as shown in FIG. 7, as a result of performing analysis after adding the anti-PD-Ll antibody to the extract from the HEK293T cell line transformed with the IVT-mRNA encoding the single or chimeric antigen according to the present invention, it could be confirmed that PD-L1 interacted with PD-1, PD-1/HBc or PD-1/HBe.

### <Example 5>

### Fabrication of Lipid Nanoparticles (LNPs) for Increasing Delivery Efficiency of IVT mRNA Encoding Chimeric Antigen

In order to increase the stability of mRNA and the efficiency of delivery thereof to cells, lipid nanoparticles (LNPs) composed of various lipids were fabricated (Cell, 2017, 168:1). First, four different lipids, SS-OP, DOPC, cholesterol and PEG-lipid, were dissolved in 100% ethanol, and then mixed together at a molar ratio of 50:10:38.5:1.5. From the single antigen or chimeric antigen-encoding IVT mRNA of the present invention, contained in 50 mM citrate buffer (pH 3.0), and the lipid mixture, LNPs were fabricated using a microfluidic device (Precision Nanosystems, CA). The fabricated LNPs were transferred into a dialysis membrane cassette, and then dialyzed with 1x PBS buffer for 18 hours and adjusted to a suitable concentration using a centrifugal filter (Merck Millipore, Germany). After adjustment of the concentration, the LNPs were filtered through a 0.22-µm filter and cold-stored at 4°C until use for intracellular injection or mouse injection.

### <Example 6>

### Measurement of Size of LNPs Encapsulated with IVT mRNA and mRNA Encapsulation Efficiency

The particle size and polydispersity index (PDI) of the LNPs fabricated in Example 5 above were measured using a Zetasizer nanoparticle analyzer (Malvern, GB).

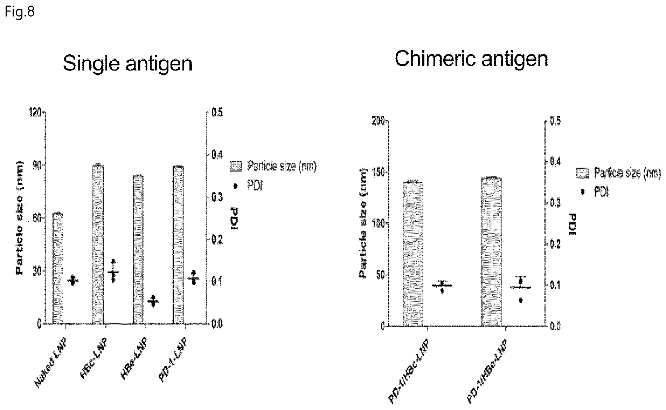

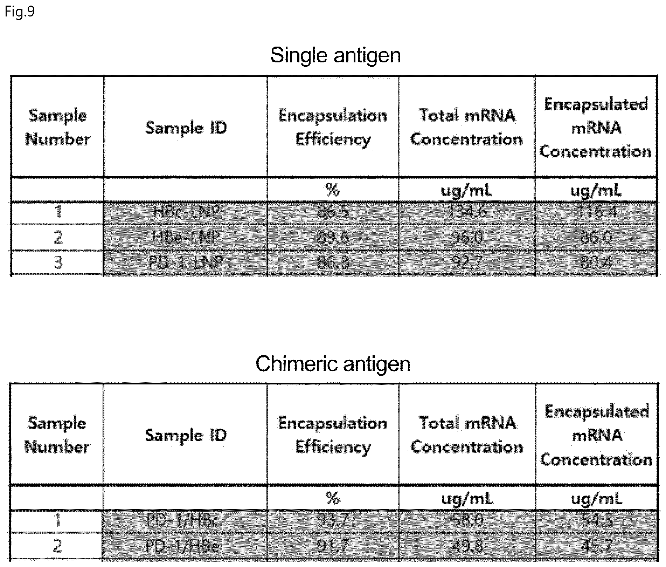

As a result, as shown in FIG. 8, the LNPs encapsulated with the IVT mRNA showed a particle size of 90 to 150 nm, which was slightly larger than that of naked LNPs not containing the IVT mRNA. In addition, the mRNA encapsulation efficiency of the IVT mRNA into LNPs in the LNP fabrication process was measured by Ribogreen assay using a Quanti-iT Ribogreen kit (Thermo Fisher Scientific, USA). As a result, as shown in FIG. 9, the encapsulation efficiency of each IVT mRNA in the process of fabricating LNPs encapsulated with each of the single antigen and chimeric antigens of the present invention was high at about 86 to 94%.

### <Example 7>

### Analysis of Antigen Expression Efficiency at Cellular Level of LNPs Encapsulated with IVT mRNA Encoding Chimeric Antigen

In order to examine whether the LNPs fabricated in Example 5 above can actually produce the protein in cells, the LNPs encapsulated with each of the single antigen HBcAg and HBeAg IVT mRNAs produced in the present invention was transfected into the human cell line HeLa (ATCC, CCL-2). HeLa is a cancer cell line originating from cervical cancer. For culture, the cancer cells were cultured for 3 days in MEM medium (GIBCO, #11095-080) (supplemented with 10% FBS (Fetus Bovine Serum: GIBCO, #11095-080), 1% ABAM (GIBCO BRL, #15240-013)) in a T75 flask, and washed with 1x PBS buffer and then detached from the flask bottom by treatment with trypsin. The isolated cells were seeded into a 24-well culture dish at a density of 5 x 10⁵ cells/well, and then cultured in a 5% CO₂ incubator for 24 hours. The next day, the LNPs of the present invention were mixed with apolipoprotein E4 (ApoE4) and incubated at 37°C for 10 minutes, and then transfection into the cells. The culture of the transfected cells was harvested, and then the amount of the antigen secreted from the cells was analyzed by enzyme-linked immunosorbent assay (ELISA).

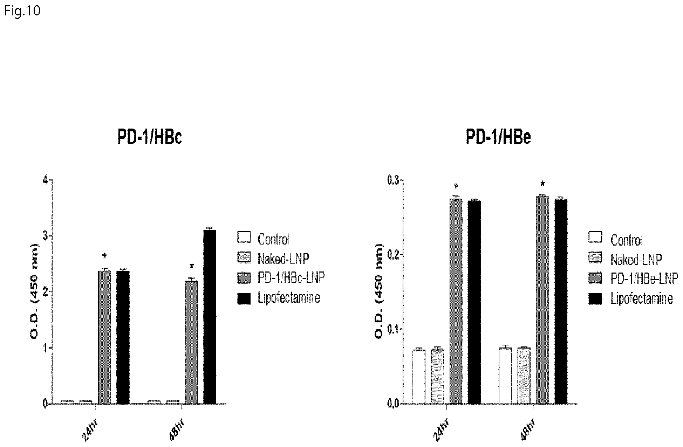

As a result, as shown in FIG. 10, it could be confirmed that the LNPs encapsulated with each of the PD-1/HBc and PD-1/HBe IVT mRNAs could efficiently produce the antigen after transfection into the HeLa cells.

### <Example 8>

### Analysis of Antigen Expression of LNPs Encapsulated with Chimeric Antigen-Encoding IVT mRNA in Mice

In addition, the present inventors confirmed the antigen expression results, confirmed at the cellular level in Example 7, through experimental animals. To this end, the LNPs fabricated in Example 5 above were injected into the left tibialis anterior of 6-week-old C56BL/6 female mice, and the expression efficiency of the antigen was analyzed, thereby analyzing the efficiency of the LNPs as an IVT mRNA vehicle for delivery into the animal body. To this end, the LNPs encapsulated with 2 µg of the IVT mRNA of the present invention were inoculated into each mouse together with 1x PBS buffer in a total volume of 80 µl. 1, 2 and 3 days after inoculation, whole blood was collected from the heart, and whether each antigen protein was produced was analyzed by enzyme-linked immunosorbent assay (ELISA).

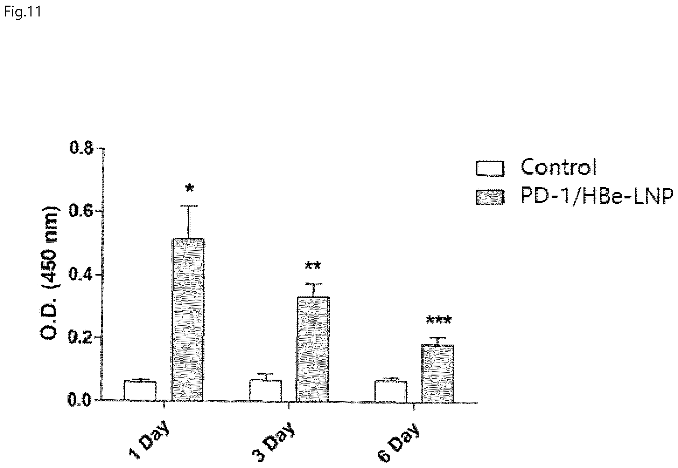

As a result, as shown in FIG. 11, it could be confirmed that the PD-1/HBe chimeric antigen was produced from day 1 to day 6, and that when the PD-1/HBe chimeric antigen was injected into the mice using the LNPs, sufficient amounts of the single antibody and chimeric antibodies were actually produced in the mouse body.

### <Example 9>

### Analysis of Formation of Antibodies against HBcAg and HBeAg Protein Antigens in Mice Inoculated with These Antigens

On day 1, each of HBcAg protein (#MBS355629, MyBioSource, USA) and HBeAg protein (#MBS355623, MyBioSource, USA) was mixed with an adjuvant (#F5881, Sigma, USA), and then 2 µg of each antigen protein was injected into the left tibialis anterior of each of 6-week-old C57BL/6 female mice three times (30 µl each). 11 days after the first inoculation, the same amount of the antigen protein was injected into each mouse in the same manner. 7, 21, 35 and 44 days after the first inoculation, serum was collected from the tail artery of each immunized mouse, and whether the antibody was produced was analyzed by enzyme-linked immunosorbent assay.

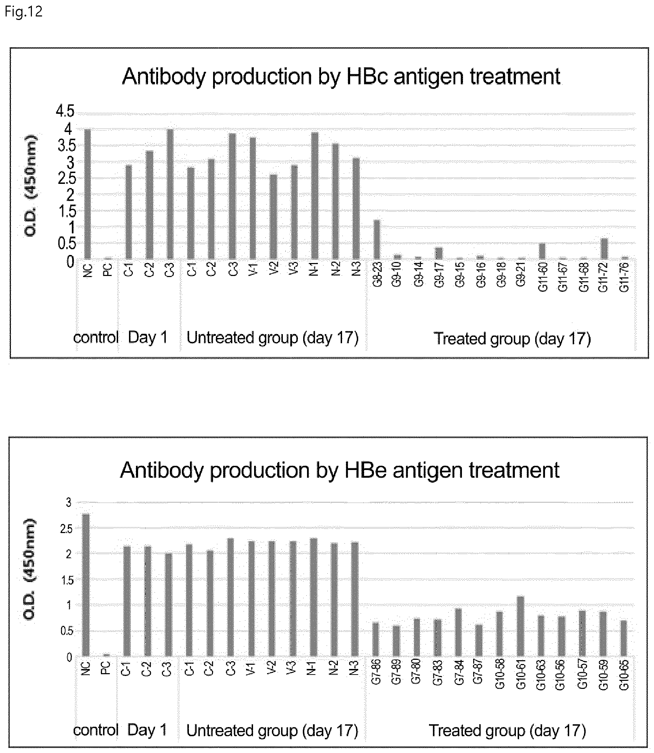

As a result, as shown in FIG. 12, it was confirmed that the antibody against each of HBcAg and HBeAg was formed in each mouse inoculated with each of the antigens.

### <Example 10>

### Evaluation of Anticancer Activity of Chimeric Antigen (PD-1/HBc or PD-1/HBe) of the Present Invention in Cancer Cells Using Mouse Xenograft Model

21 days after inoculation with each antigen, about 1x10⁶ melanoma B16F10 cancer cells were injected subcutaneously into the lateral side of each C57BL/6 mouse having the antibody formed by inoculation with each of the HBcAg and HBeAg antigen proteins in Example 9. 10 days after administration of the cancer cells, the IVT mRNA-LNPs encoding each of the chimeric antigens PD-1/HBc and PD-1/HBe of the present invention was injected (main inoculation) into the left tibialis anterior of each mouse. Every 3 days after injection of the cancer cells, changes in the mouse body weight and the formation of cancer tissue in the mice were observed, and the size of cancer tissue in the mice was measured. At this time, the number of the mice used in the experiment was 6 to 7 for each test group.

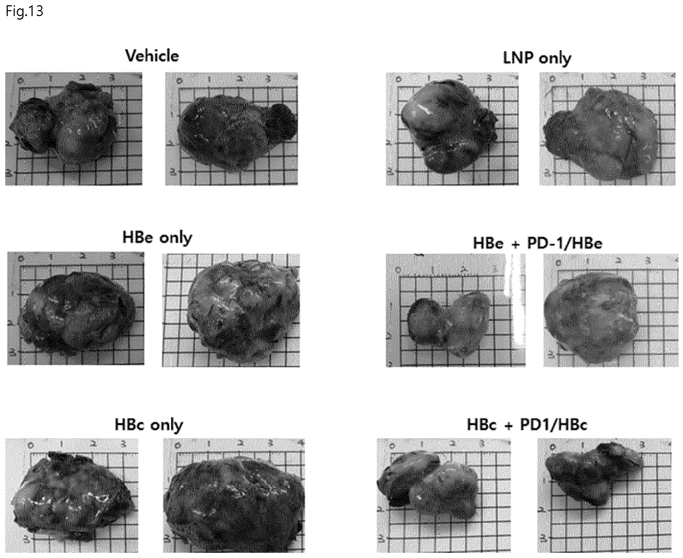

As a result, as shown in FIG. 13, it was confirmed that the growth of cancer tissue in the mice pre-inoculated with the HBcAg antigen was significantly inhibited compared to that in the control group, when main inoculation with the chimeric antigen PD-1/HBc IVT mRNA-LNP of the present invention was performed, and likewise, the growth of cancer tissue in the mice pre-inoculated with the HBeAg antigen was significantly inhibited when main inoculation with the chimeric antigen PD-1/HBe IVT mRNA-LNP of the present invention was performed.

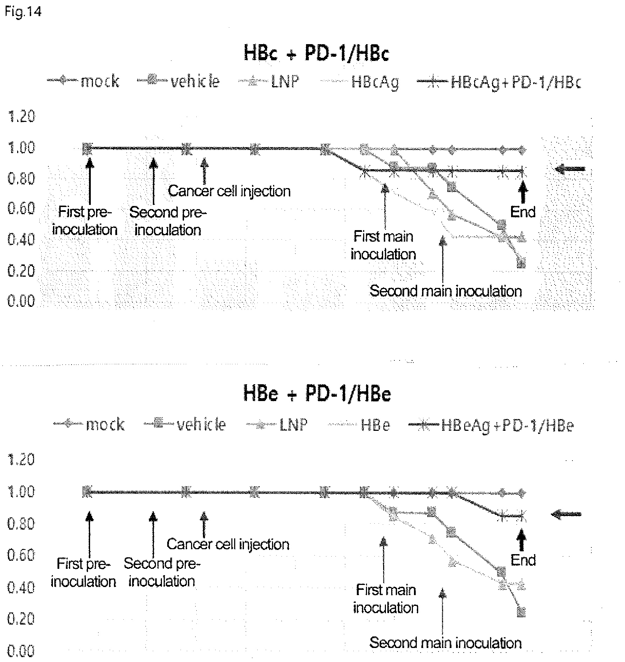

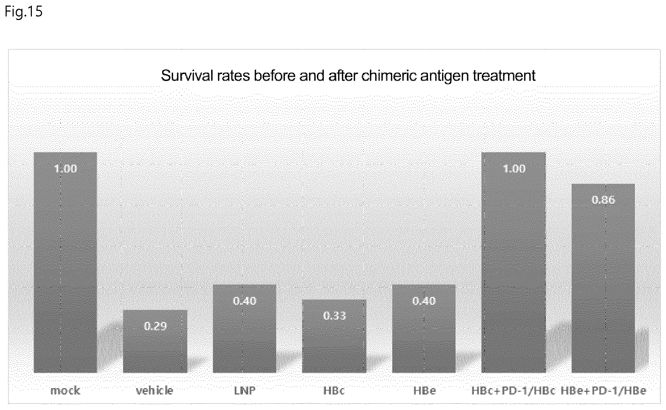

In addition, as shown in FIG. 14, it could be confirmed that the mortality rate of the mice in the test group inoculated with each of the chimeric antigens PD-1/HBc and PD-1/HBe IVT mRNA-LNPs of the present invention significantly increased during the 44-day test period compared to that in the test group, and the survival rate thereof increased about 2 times. It can be seen that this decrease in mouse mortality is due to injection of the chimeric antigen of the present invention. As a result of comparing the survival rate of the mice before and after inoculation with the chimeric antigen of the present invention, it could be confirmed that the survival rate significantly increased only in the test group inoculated with the chimeric antigen of the present invention (FIG. 15) .

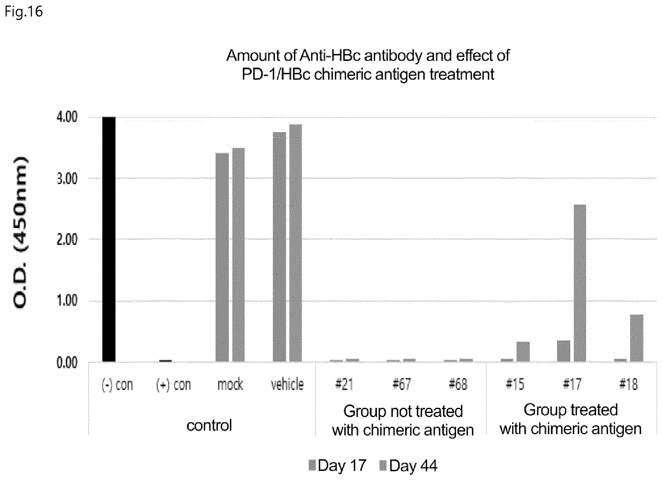

In addition, FIG. 16 shows the results obtained by forming the antibody against the HBcAg antigen in C57BL/6 mice, transplanting melanoma cancer B16F10 cells into the mice to induce the production of cancer tissue, injecting, into the mice, LNPs encapsulated with the IVT mRNA encoding PD-1/HBc, and examining the relationship between the relative amount of the antibody to the HBcAg antigen and the size of the cancer tissue before and after injection of the LNPs. As shown therein, the amount of the antibody against the HBcAg antigen slightly decreased in the mice in which the size of the cancer tissue significantly decreased. This is believed to be because the antibody against the HBcAg antigen was consumed during attachment to natural killer cells in the process in which the growth of the cancer tissue was inhibited by the antibody-dependent cellular cytotoxicity (ADCC) of the antibody against the HBcAg antigen in the mouse body. These results prove the inventor's initial prediction that the chimeric antigen inhibits the growth of cancer tissue by antibody-dependent humoral immunity.

In conclusion, the present inventors found that, when a chimeric antigen obtained by fusing a peptide domain, which is capable of binding to a disease-related target protein, to a specific foreign antigen capable of triggering a specific antigen-antibody reaction system already formed in the body, is administered, the chimeric antigen may bind specifically to cancer or infected cells having the target protein on the cell membrane, and at the same time, may trigger the antigen-antibody reaction system, already formed in the body, by the other domain of the chimeric antigen, thereby effectively removing target diseased cells, thereby treating cancer or infectious disease and greatly increasing the survival of the patient. Therefore, the chimeric antigen may provide a new method for treating cancer or infectious disease.

So far, the present invention has been described with reference to the preferred embodiments thereof. Those skilled in the art will appreciate that the present invention can be implemented in modified forms without departing from the essential features of the present invention. Therefore, the disclosed embodiments should be considered in a descriptive sense only and not for purposes of limitation. Therefore, the scope of the present invention is defined not by the detailed description of the present disclosure but by the appended claims, and all modifications within a range equivalent to the scope of the appended claims should be construed as being included in the present invention.

## Claims

1. A chimeric antigen for enhancing multiple immune functions wherein an immune response-inducing domain and a domain for inducing target cell-specific binding are fused to each other.

2. The chimeric antigen of claim 1, wherein the immune response-inducing domain is a foreign viral antigen, and an antigen-antibody reaction is formed by the antigen.

3. The chimeric antigen of claim 2, wherein the immune response-inducing domain is selected from the group consisting of hepatitis B virus antigen, hepatitis C virus antigen, hepatitis A virus antigen, AIDS virus antigen, MERS virus antigen, influenza A virus antigen, human papillomavirus antigen, lymphocytic choriomeningitis virus (LCMV) antigen, and herpes simplex virus (HSV) antigen.

4. The chimeric antigen of claim 3, wherein the immune response-inducing domain is hepatitis B virus core protein (HBc), hepatitis B virus e antigen (HBe), or hepatitis B virus surface protein (HBs).

5. The chimeric antigen of claim 1, wherein the domain for inducing target cell-specific binding is a cell membrane protein capable of binding specifically to target cells, and is selected from the group consisting of PD-1, PD-L1, PD-L2, CTLA-4, CD28, ICOS, OX40, OX40L, GITR, GITRL, CD40, CD40L, 4-1BB, 4-1BBL, B7-1, B7-2, B7-H1, B7-H2, B7-DC, CD80, CD160, BTLA, HVEM, DPP-4, NTCP, CD16, and Caveolin-1.

6. The chimeric antigen of claim 1, wherein the immune response-inducing domain and the domain for inducing target cell-specific binding are linked to each other by a linker peptide represented by SEQ ID NO: 19 or SEQ ID NO: 21 to increase structural stability of the chimeric antigen.

7. The chimeric antigen of claim 1, wherein the chimeric antigen for enhancing multiple immune functions consists of the peptide sequence of SEQ ID NO: 9 or SEQ ID NO: 11.

8. A pharmaceutical composition for preventing or treating cancer, the pharmaceutical composition containing, as an active ingredient, the chimeric antigen for enhancing multiple immune functions according to claim 1.

9. The pharmaceutical composition of claim 8, wherein the cancer is selected from the group consisting of lung cancer, stomach cancer, liver cancer, bone cancer, pancreatic cancer, skin cancer, head and neck cancer, skin melanoma, uterine cancer, ovarian cancer, rectal cancer, colorectal cancer, colon cancer, breast cancer, uterine sarcoma, fallopian tube carcinoma, endometrial carcinoma, cervical carcinoma, vaginal carcinoma, vulvar carcinoma, esophageal cancer, laryngeal cancer, small intestine cancer, thyroid cancer, parathyroid cancer, soft tissue sarcoma, urethral cancer, penile cancer, prostate cancer, chronic or acute leukemia, childhood solid tumor, differentiated lymphoma, bladder cancer, kidney cancer, renal cell carcinoma, renal pelvic carcinoma, primary central nervous system lymphoma, spinal cord tumor, brainstem glioma, and pituitary adenoma.

10. A pharmaceutical composition for preventing or treating infectious disease, the pharmaceutical composition containing, as an active ingredient, the chimeric antigen for enhancing multiple immune functions according to claim 1.

11. The pharmaceutical composition of claim 10, wherein the infectious disease is hepatitis B virus, hepatitis C virus, hepatitis A virus, AIDS virus, MERS virus, influenza A virus, human papillomavirus, lymphocytic choriomeningitis virus (LCMV) or herpes simplex virus (HSV) infection.

12. A composition for enhancing immunity containing, as an active ingredient, the chimeric antigen for enhancing multiple immune functions according to claim 1.

13. The composition of claim 12, wherein the chimeric antigen for enhancing multiple immune functions
(i) binds specifically to diseased cells which are target cells;
(ii) induces T cell activation by stimulating restoration of exhausted CD8+ T cells; and
(iii) enhances humoral immune response and activates cytotoxicity of natural killer (NK) cells by inducing an antigen-antibody reaction.

14. The composition of claim 13, wherein the inducing the antigen-antibody reaction in (iii) comprises enhancing a specific antigen-antibody reaction, formed in a body of a subject, by the immune response-inducing domain of the chimeric antigen.

15. A vaccine composition containing, as an active ingredient, the chimeric antigen for enhancing multiple immune functions according to claim 1.
